# EUROPEAN PATENT SPECIFICATION

(11) **EP 2 275 531 B1**
(45) Date of publication and mention of the grant of the patent: **23.12.2015**
(21) Application number: 09728541.5
(22) Date of filing: 31.03.2009
(51) Int. Cl.: C07K 14/47, C12N 15/12, C12N 5/0735

(54) **METHOD FOR PROLIFERATION OF PLURIPOTENT STEM CELLS**
VERFAHREN ZUR VERMEHRUNG PLURIPOTENTER STAMMZELLEN
PROCÉDÉ POUR LA PROLIFÉRATION DE CELLULES SOUCHES PLURIPOTENTES

(30) Priority: 31.03.2008 JP 2008093350; 03.09.2008 JP 2008225686
(43) Date of publication of application: 19.01.2011
(73) Proprietor: Oriental Yeast Co., Ltd., Tokyo 174-8505 (JP); Kyoto University, Sakyo-ku Kyoto-shi Kyoto 606-8501 (JP)
(72) Inventor: YASUDA, Hisataka, Nagahama-shi Shiga 5260804 (JP); YAMADA, Munehiro, Nagahama-shi Shiga 526-0804 (JP); MIYAZAKI, Kaoru, Fujisawa-shi Kanagawa 251-0861 (JP); TAKAHASHI, Kazutoshi, Kyoto-shi Kyoto 606-8507 (JP)
(74) Representative: Margue, Robert Germain
(86) International application number: PCT/JP2009/057041
(87) International publication number: WO 2009/123349

(56) References cited:
- WO-A1-98/30679
- WO-A1-98/30679
- WO-A1-2007/023875
- WO-A2-2006/029198
- WO-A2-2006/029198
- WO-A2-2008/084401
- US-A1- 2005 233 446
- DOMOGATSKAYA ANNA ET AL: "Laminin-511 but not -332, -111, or -411 enables mouse embryonic stem cell self-renewal in vitro", STEM CELLS, ALPHAMED PRESS, INC, UNITED STATES, vol. 26, no. 11, 1 November 2008 (2008-11-01), pages 2800-2809, XP009109423, ISSN: 1549-4918, DOI: 10.1634/STEMCELLS.2007-0389
- HASHIMOTO JUNKO ET AL: "Regulation of proliferation and chondrogenic differentiation of human mesenchymal stem cells by laminin-5 (laminin-332)", STEM CELLS, ALPHAMED PRESS, DAYTON, OH, US, vol. 24, no. 11, 1 November 2006 (2006-11-01), pages 2346-2354, XP002506193, ISSN: 1066-5099, DOI: 10.1634/STEMCELLS.2005-0605
- KLEES ET AL: "Laminin-5 activates extracellular matrix production and osteogenic gene focusing in human mesenchymal stem cells", MATRIX BIOLOGY, ELSEVIER, NL, vol. 26, no. 2, 3 February 2007 (2007-02-03), pages 106-114, XP005872205, ISSN: 0945-053X, DOI: 10.1016/J.MATBIO.2006.10.001
- HASHIMOTO J ET AL.: 'Regulation of proliferation and chondrogenic differentiation of human mesenchymal stem cells by laminin-5 (laminin-322).' STEM CELLS vol. 24, 2006, pages 2346 - 2354, XP002506193
- LI Y ET AL.: 'Expansion of human embryonic stem cells in defined serum-free medium devoid of animal-derived products.' BIOTECHNOL. BIOENG. vol. 91, 2005, pages 688 - 698, XP002351170
- KLIMANSKAYA I ET AL.: 'Human embryonic stem cells derived without feeder cells.' LANCET vol. 365, 2005, pages 1636 - 1641, XP025276910
- DRAPER J S ET AL.: 'Culture and characterization of human embryonic stem cells.' STEM CELLS AND DEVELOPMENT vol. 13, 2004, pages 325 - 336, XP002405631

### SEQUENCE LISTING

<110> Oriental Yeast Co., Ltd.
<120> METHOD FOR PROLIFERATION OF PLURIPOTENT STEM CELLS
<130> FA1312-09040
<150> JP2008-093350
   <151> 2008-03-31
<150> JP2008-225686
   <151> 2008-09-03
<160> 42
<210> 1
   <211> 5433
   <212> DNA
   <213> Human
<220>
   <221> CDS
   <222> (1)...(5139)
<300>
   <301> Ryan,M.C., Tizard,R., VanDevanter,D.R. and Carter, W.G.
   <302> Cloning of the LamA3 gene encoding the alpha 3 chain of the adhesive ligand epiligrin. Expression in wound repair
   <303> JOURNAL J. Biol. Chem.
   <304> 269
   <305> 36
   <306> 22779-22787
   <307> 1994
<400> 1
<210> 2
   <211> 1713
   <212> PRT
   <213> Human
<400> 2
   Met Gly Trp Leu Trp Ile Phe Gly Ala Ala Leu Gly Gln Cys Leu
   1               5                   10                  15
   Gly Tyr Ser Ser Gln Gln Gln Arg Val Pro Phe Leu Gln Pro Pro
                   20                  25                  30
   Gly Gln Ser Gln Leu Gln Ala Ser Tyr Val Glu Phe Arg Pro Ser
                   35                  40                  45
   Gln Gly Cys Ser Pro Gly Tyr Tyr Arg Asp His Lys Gly Leu Tyr
                   50                  55                  60
   Thr Gly Arg Cys Val Pro Cys Asn Cys Asn Gly His Ser Asn Gln
                   65                  70                  75
   Cys Gln Asp Gly Ser Gly Ile Cys Val Asn Cys Gln His Asn Thr
                   80                  85                  90
   Ala Gly Glu His Cys Glu Arg Cys Gln Glu Gly Tyr Tyr Gly Asn
                   95                  100                 105
   Ala Val His Gly Ser Cys Arg Ala Cys Pro Cys Pro His Thr Asn
                   110                 115                 120
   Ser Phe Ala Thr Gly Cys Val Val Asn Gly Gly Asp Val Arg Cys
                   125                 130                 135
   Ser Cys Lys Ala Gly Tyr Thr Gly Thr Gln Cys Glu Arg Cys Ala
                   140                 145                 150
   Pro Gly Tyr Phe Gly Asn Pro Gln Lys Phe Gly Gly Ser Cys Gln
                   155                 160                 165
   Pro Cys Ser Cys Asn Ser Asn Gly Gln Leu Gly Ser Cys His Pro
                   170                 175                 180
   Leu Thr Gly Asp Cys Ile Asn Gln Glu Pro Lys Asp Ser Ser Pro
                   185                 190                 195
   Ala Glu Glu Cys Asp Asp Cys Asp Ser Cys Val Met Thr Leu Leu
                       200                     205                     210
   Asn Asp Leu Ala Thr Met Gly Glu Gln Leu Arg Leu Val Lys Ser
                       215                     220                     225
   Gln Leu Gln Gly Leu Ser Ala Ser Ala Gly Leu Leu Glu Gln Met
                       230                     235                     240
   Arg His Met Glu Thr Gln Ala Lys Asp Leu Arg Asn Gln Leu Leu
                       245                     250                     255
   Asn Tyr Arg Ser Ala Ile Ser Asn His Gly Ser Lys Ile Glu Gly
                       260                     265                     270
   Leu Glu Arg Glu Leu Thr Asp Leu Asn Gln Glu Phe Glu Thr Leu
                       275                     280                     285
   Gln Glu Lys Ala Gln Val Asn Ser Arg Lys Ala Gln Thr Leu Asn
                       290                     295                     300
   Asn Asn Val Asn Arg Ala Thr Gln Ser Ala Lys Glu Leu Asp Val
                       305                     310                     315
   Lys Ile Lys Asn Val Ile Arg Asn Val His Ile Leu Leu Lys Gln
                       320                     325                     330
   Ile Ser Gly Thr Asp Gly Glu Gly Asn Asn Val Pro Ser Gly Asp
                       335                     340                     345
   Phe Ser Arg Glu Trp Ala Glu Ala Gln Arg Met Met Arg Glu Leu
                       350                     355                     360
   Arg Asn Arg Asn Phe Gly Lys His Leu Arg Glu Ala Glu Ala Asp
                       365                     370                     375
   Lys Arg Glu Ser Gln Leu Leu Leu Asn Arg Ile Arg Thr Trp Gln
                       380                     385                     390
   Lys Thr His Gln Gly Glu Asn Asn Gly Leu Ala Asn Ser Ile Arg
                       395                     400                     405
   Asp Ser Leu Asn Glu Tyr Glu Ala Lys Leu Ser Asp Leu Arg Ala
                       410                     415                     420
   Arg Leu Gln Glu Ala Ala Ala Gln Ala Lys Gln Ala Asn Gly Leu
                       425                     430                     435
   Asn Gln Glu Asn Glu Arg Ala Leu Gly Ala Ile Gln Arg Gln Val
                       440                     445                     450
   Lys Glu Ile Asn Ser Leu Gln Ser Asp Phe Thr Lys Tyr Leu Thr
                       455                     460                     465
   Thr Ala Asp Ser Ser Leu Leu Gln Thr Asn Ile Ala Leu Gln Leu
                       470                     475                     480
   Met Glu Lys Ser Gln Lys Glu Tyr Glu Lys Leu Ala Ala Ser Leu
                       485                     490                     495
   Asn Glu Ala Arg Gln Glu Leu Ser Asp Lys Val Arg Glu Leu Ser
                       500                     505                     510
   Arg Ser Ala Gly Lys Thr Ser Leu Val Glu Glu Ala Glu Lys His
                       515                     520                     525
   Ala Arg Ser Leu Gln Glu Leu Ala Lys Gln Leu Glu Glu Ile Lys
                       530                     535                     540
   Arg Asn Ala Ser Gly Asp Glu Leu Val Arg Cys Ala Val Asp Ala
                       545                     550                     555
   Ala Thr Ala Tyr Glu Asn Ile Leu Asn Ala Ile Lys Ala Ala Glu
                       560                     565                     570
   Asp Ala Ala Asn Arg Ala Ala Ser Ala Ser Glu Ser Ala Leu Gln
                       575                     580                     585
   Thr Val Ile Lys Glu Asp Leu Pro Arg Lys Ala Lys Thr Leu Ser
                       590                     595                     600
   Ser Asn Ser Asp Lys Leu Leu Asn Glu Ala Lys Met Thr Gln Lys
                       605                     610                     615
   Lys Leu Lys Gln Glu Val Ser Pro Ala Leu Asn Asn Leu Gln Gln
                       620                     625                     630
   Thr Leu Asn Ile Val Thr Val Gln Lys Glu Val Ile Asp Thr Asn
                       635                     640                     645
   Leu Thr Thr Leu Arg Asp Gly Leu His Gly Ile Gln Arg Gly Asp
                       650                     655                     660
   Ile Asp Ala Met Ile Ser Ser Ala Lys Ser Met Val Arg Lys Ala
                       665                     670                     675
   Asn Asp Ile Thr Asp Glu Val Leu Asp Gly Leu Asn Pro Ile Gln
                       680                     685                     690
   Thr Asp Val Glu Arg Ile Lys Asp Thr Tyr Gly Arg Thr Gln Asn
                       695                     700                     705
   Glu Asp Phe Lys Lys Ala Leu Thr Asp Ala Asp Asn Ser Val Asn
                       710                     715                     720
   Lys Leu Thr Asn Lys Leu Pro Asp Leu Trp Arg Lys Ile Glu Ser
                       725                     730                     735
   Ile Asn Gln Gln Leu Leu Pro Leu Gly Asn Ile Ser Asp Asn Met
                       740                     745                     750
   Asp Arg Ile Arg Glu Leu Ile Gln Gln Ala Arg Asp Ala Ala Ser
                       755                     760                     765
   Lys Val Ala Val Pro Met Arg Phe Asn Gly Lys Ser Gly Val Glu
   770 775 780
   Val Arg Leu Pro Asn Asp Leu Glu Asp Leu Lys Gly Tyr Thr Ser
   785 790 795
   Leu Ser Leu Phe Leu Gln Arg Pro Asn Ser Arg Glu Asn Gly Gly
   800 805 810
   Thr Glu Asn Met Phe Val Met Tyr Leu Gly Asn Lys Asp Ala Ser
   815 820 825
   Arg Asp Tyr Ile Gly Met Ala Val Val Asp Gly Gln Leu Thr Cys
   830 835 840
   Val Tyr Asn Leu Gly Asp Arg Glu Ala Glu Leu Gln Val Asp Gln
   845 850 855
   Ile Leu Thr Lys Ser Glu Thr Lys Glu Ala Val Met Asp Arg Val
   860 865 870
   Lys Phe Gln Arg Ile Tyr Gln Phe Ala Arg Leu Asn Tyr Thr Lys
   875 880 885
   Gly Ala Thr Ser Ser Lys Pro Glu Thr Pro Gly Val Tyr Asp Met
   890 895 900
   Asp Gly Arg Asn Ser Asn Thr Leu Leu Asn Leu Asp Pro Glu Asn
   905 910 915
   Val Val Phe Tyr Val Gly Gly Tyr Pro Pro Asp Phe Lys Leu Pro
   920 925 930
   Ser Arg Leu Ser Phe Pro Pro Tyr Lys Gly Cys Ile Glu Leu Asp
   935 940 945
   Asp Leu Asn Glu Asn Val Leu Ser Leu Tyr Asn Phe Lys Lys Thr
   950 955 960
   Phe Asn Leu Asn Thr Thr Glu Val Glu Pro Cys Arg Arg Arg Lys
   965 970 975
   Glu Glu Ser Asp Lys Asn Tyr Phe Glu Gly Thr Gly Tyr Ala Arg
   980 985 990
   Val Pro Thr Gln Pro His Ala Pro Ile Pro Thr Phe Gly Gln Thr
   995 1000 1005
   Ile Gln Thr Thr Val Asp Arg Gly Leu Leu Phe Phe Ala Glu Asn
   1010 1015 1020
   Gly Asp Arg Phe Ile Ser Leu Asn Ile Glu Asp Gly Lys Leu Met
   1025 1030 1035
   Val Arg Tyr Lys Leu Asn Ser Glu Leu Pro Lys Glu Arg Gly Val
   1040 1045 1050
   Gly Asp Ala Ile Asn Asn Gly Arg Asp His Ser Ile Gln Ile Lys
   1055 1060 1065
   Ile Gly Lys Leu Gln Lys Arg Met Trp Ile Asn Val Asp Val Gln
   1070 1075 1080
   Asn Thr Ile Ile Asp Gly Glu Val Phe Asp Phe Ser Thr Tyr Tyr
   1085 1090 1095
   Leu Gly Gly Ile Pro Ile Ala Ile Arg Glu Arg Phe Asn Ile Ser
   1100 1105 1110
   Thr Pro Ala Phe Arg Gly Cys Met Lys Asn Leu Lys Lys Thr Ser
   1115 1120 1125
   Gly Val Val Arg Leu Asn Asp Thr Val Gly Val Thr Lys Lys Cys
   1130 1135 1140
   Ser Glu Asp Trp Lys Leu Val Arg Ser Ala Ser Phe Ser Arg Gly
   1145 1150 1155
   Gly Gln Leu Ser Phe Thr Asp Leu Gly Leu Pro Pro Thr Asp His
   1160 1165 1170
   Leu Gln Ala Ser Phe Gly Phe Gln Thr Phe Gln Pro Ser Gly Ile
   1175 1180 1185
   Leu Leu Asp His Gln Thr Trp Thr Arg Asn Leu Gln Val Thr Leu
   1190 1195 1200
   Glu Asp Gly Tyr Ile Glu Leu Ser Thr Ser Asp Ser Gly Gly Pro
   1205 1210 1215
   Ile Phe Lys Ser Pro Gln Thr Tyr Met Asp Gly Leu Leu His Tyr
   1220 1225 1230
   Val Ser Val Ile Ser Asp Asn Ser Gly Leu Arg Leu Leu Ile Asp
   1235 1240 1245
   Asp Gln Leu Leu Arg Asn Ser Lys Arg Leu Lys His Ile Ser Ser
   1250 1255 1260
   Ser Arg Gln Ser Leu Arg Leu Gly Gly Ser Asn Phe Glu Gly Cys
   1265 1270 1275
   Ile Ser Asn Val Phe Val Gln Arg Leu Ser Leu Ser Pro Glu Val
   1280 1285 1290
   Leu Asp Leu Thr Ser Asn Ser Leu Lys Arg Asp Val Ser Leu Gly
   1295 1300 1305
   Gly Cys Ser Leu Asn Lys Pro Pro Phe Leu Met Leu Leu Lys Gly
   1310 1315 1320
   Ser Thr Arg Phe Asn Lys Thr Lys Thr Phe Arg Ile Asn Gln Leu
   1325 1330 1335
   Leu Gln Asp Thr Pro Val Ala Ser Pro Arg Ser Val Lys Val Trp
                   1340                1345                1350
   Gln Asp Ala Cys Ser Pro Leu Pro Lys Thr Gln Ala Asn His Gly
                   1355                1360                1365
   Ala Leu Gln Phe Gly Asp Ile Pro Thr Ser His Leu Leu Phe Lys
                   1370                1375                1380
   Leu Pro Gln Glu Leu Leu Lys Pro Arg Ser Gln Phe Ala Val Asp
                   1385                1390                1395
   Met Gln Thr Thr Ser Ser Arg Gly Leu Val Phe His Thr Gly Thr
                   1400                1405                1410
   Lys Asn Ser Phe Met Ala Leu Tyr Leu Ser Lys Gly Arg Leu Val
                   1415                1420                1425
   Phe Ala Leu Gly Thr Asp Gly Lys Lys Leu Arg Ile Lys Ser Lys
                   1430                1435                1440
   Glu Lys Cys Asn Asp Gly Lys Trp His Thr Val Val Phe Gly His
                   1445                1450                1455
   Asp Gly Glu Lys Gly Arg Leu Val Val Asp Gly Leu Arg Ala Arg
                   1460                1465                1470
   Glu Gly Ser Leu Pro Gly Asn Ser Thr Ile Ser Ile Arg Ala Pro
                   1475                1480                1485
   Val Tyr Leu Gly Ser Pro Pro Ser Gly Lys Pro Lys Ser Leu Pro
                   1490                1495                1500
   Thr Asn Ser Phe Val Gly Cys Leu Lys Asn Phe Gln Leu Asp Ser
                   1505                1510                1515
   Lys Pro Leu Tyr Thr Pro Ser Ser Ser Phe Gly Val Ser Ser Cys
                   1520                1525                1530
   Leu Gly Gly Pro Leu Glu Lys Gly Ile Tyr Phe Ser Glu Glu Gly
                   1535                1540                1545
   Gly His Val Val Leu Ala His Ser Val Leu Leu Gly Pro Glu Phe
                   1550                1555                1560
   Lys Leu Val Phe Ser Ile Arg Pro Arg Ser Leu Thr Gly Ile Leu
                   1565                1570                1575
   Ile His Ile Gly Ser Gln Pro Gly Lys His Leu Cys Val Tyr Leu
                   1580                1585                1590
   Glu Ala Gly Lys Val Thr Ala Ser Met Asp Ser Gly Ala Gly Gly
                   1595                1600                1605
   Thr Ser Thr Ser Val Thr Pro Lys Gln Ser Leu Cys Asp Gly Gln
                   1610                1615                1620
   Trp His Ser Val Ala Val Thr Ile Lys Gln His Ile Leu His Leu
                   1625                1630                1635
   Glu Leu Asp Thr Asp Ser Ser Tyr Thr Ala Gly Gln Ile Pro Phe
                   1640                1645                1650
   Pro Pro Ala Ser Thr Gln Glu Pro Leu His Leu Gly Gly Ala Pro
                   1655                1660                1665
   Ala Asn Leu Thr Thr Leu Arg Ile Pro Val Trp Lys Ser Phe Phe
                   1670                1675                1680
   Gly Cys Leu Arg Asn Ile His Val Asn His Ile Pro Val Pro Val
                   1685                1690                1695
   Thr Glu Ala Leu Glu Val Gln Gly Pro Val Ser Leu Asn Gly Cys
                   1700                1705                1710
   Pro Asp Gln
           1713
<210> 3
   <211> 3930
   <212> DNA
   <213> Human
<220>
   <221> CDS
   <222> (121)...(3630)
<300>
   <301> Gerecke,D.R., Wagman, D. W., Champliaud,M.F. and Burgeson, R. E. <302> The complete primary structure for a novel laminin chain, the laminin Blk chain <303> J. Biol. Chem. <304> 269 <305> 15 <306> 11073-11080 <307> 1994
<400> 3
   gggcgggagg aggactgtat ctctggatgc ctggggcctg gtttcagggc ctgatttatt 60
   cctcttcctg ggagctcact caggaaaggt cctttctggg gatcacccca ttggctgaag 120
   atgagaccat tcttcctctt gtgttttgcc ctgcctggcc tcctgcatgc ccaacaagcc 180
   tgctcccgtg gggcctgcta tccacctgtt ggggacctgc ttgttgggag gacccggttt 240
   ctccgagctt catctacctg tggactgacc aagcctgaga cctactgcac ccagtatggc 300
   gagtggcaga tgaaatgctg caagtgtgac tccaggcagc ctcacaacta ctacagtcac 360
   cgagtagaga atgtggcttc atcctccggc cccatgcgct ggtggcagtc ccagaatgat 420
   gtgaaccctg tctctctgca gctggacctg gacaggagat tccagcttca agaagtcatg 480
   atggagttcc gagggcccat gcctgccggc atgctgattg agcgctcctc agacttcggt 540
   aagacctggc gagtgtacca gtacctggct gccgactgca cctccacctt ccctcgggtc 600
   cgccagggtc ggcctcagag ctggcaggat gttcggtgcc agtccctgcc tcagaggcct 660
   aatgcacgcc taaatggggg gaaggtccaa cttaacctta tggatttagt gtctgggatt 720
   ccagcaactc aaagtcaaaa aattcaagag gtgggggaga tcacaaactt gagagtcaat 780
   ttcaccaggc tggcccctgt gccccaaagg ggctaccacc ctcccagcgc ctactatgct 840
   gtgtcccagc tccgtctgca ggggagctgc ttctgtcacg gccatgctga tcgctgcgca 900
   cccaagcctg gggcctctgc aggctccacc gctgtgcagg tccacgatgt ctgcgtctgc 960
   cagcacaaca ctgccggccc aaattgtgag cgctgtgcac ccttctacaa caaccggccc 1020
   tggagaccgg cggagggcca ggacgcccat gaatgccaaa ggtgcgactg caatgggcac 1080
   tcagagacat gtcactttga ccccgctgtg tttgccgcca gccagggggc atatggaggt 1140
   gtgtgtgaca attgccggga ccacaccgaa ggcaagaact gtgagcggtg tcagctgcac 1200
   tatttccgga accggcgccc gggagcttcc attcaggaga cctgcatctc ctgcgagtgt 1260
   gatccggatg gggcagtcgc aggggctccc tgtgacccag tgaccgggca gtgtgtgtgc 1320
   aaggagcatg tgcagggaga gcgctgtgac ctatgcaagc cgggcttcac tggactcacc 1380
   tacgccaacc cgcgacggtg ccaccgctgt gactgcaaca tcctggggtc ccgggagatg 1440
   ccgtgtgacg aggagagtgg gcgctgcctt tgtctgccca acgtggtggg tcccaaatgt 1500
   gaccagtgtg ctccctacca ctggaagctg gccagtggcc agggctgtga accgtgtgcc 1560
   tgcgacccgc acaactccct cagcccacag tgcaaccagt tcacagggca gtgcccctgt 1620
   cgggaaggct ttggtggcct gatgtgcagc gctgcagcca tccgccagtg tccagaccgg 1680
   acctatggag acgtggccac aggatgccga gcctgtgact gtgatttccg gggaacagag 1740
   ggcccgggct gcgacaaggc atcaggccgc tgcctctgcc gccctggctt gaccgggccc 1800
   cgctgtgacc agtgccagcg aggctactgc aatcgctacc cggtgtgcgt ggcctgccac 1860
   ccttgcttcc agacctatga tgcggacctc cgggagcagg ccctgcgctt tggtagactc 1920
   ccgaatgcca ccgccagcct gtggtcaggg cctgggctgg aggaccgtgg cctggcctcc 1980
   cggatcctag atgcaaagag taagattgag cagatccgag cagttctcag cagccccgca 2040
   gtcacagagc aggaggtggc tcaggtggcc agtgccatcc tctccctcag gcgaactctc 2100
   cagggcctgc agctggatct gcccctggag gaggagacgt tgtcccttcc gagagacctg 2160
   gagagtcttg acagaagctt caatggtctc cttactatgt atcagaggaa gagggagcag 2220
   tttgaaaaaa taagcagtgc tgatccttca ggagccttcc ggatgctgag cacagcctac 2280
   gagcagtcag cccaggctgc tcagcaggtc tccgacagct cgcgcctttt ggaccagctc 2340
   agggacagcc ggagagaggc agagaggctg gtgcggcagg cgggaggagg aggaggcacc 2400
   ggcagcccca agcttgtggc cctgaggttg gagatgtctt cgttgcctga cctgacaccc 2460
   accttcaaca agctctgtgg caactccagg cagatggctt gcaccccaat atcatgccct 2520
   ggtgagctat gtccccaaga caatggcaca gcctgtgcgt cccgctgcag ggtgtccttt 2580
   cccagggccg gtggggcctt cttgatggcg gggcaggtgg ctgagcagct gcggggcttc 2640
   aatgcccagc tccagcggac caggcagatg attagggcag ccgaggaatc tgcctcacag 2700
   attcaatcca gtgcccagcg cttggagacc caggtgagcg ccagccgctc ccagatggag 2760
   gaagatgtca gacgcacacg gctcctaatc cagcaggtcc gggacttcct aacagacccc 2820
   gacactgatg cagccactat ccaggaggtc agcgaggccg tgctggccct gtggctgccc 2880
   acagactcag ctactgttct gcagaagatg aatgagatcc aggccattgc agccaggctc 2940
   cccaacgtgg acttggtgct gtcccagacc aagcaggaca ttgcgcgtgc ccgccggttg 3000
   caggctgagg ctgaggaagc caggagccga gcccatgcag tggagggcca ggtggaggat 3060
   gtggttggga acctgcggca ggggacagtg gcactgcagg aagctcagga caccatgcaa 3120
   ggcaccagcc ggtcccttcg gcttatccag gacagggttg ctgaggttca gcaggtactg 3180
   cggccagcag aaaagctggt gacaagcatg accaagcagc tgggtgactt ctggacacgg 3240
   atggaggagc tccgccacca agcccggcag cagggggcag aggcagtcca ggcccagcag 3300
   cttgcggaag gtgccagcga gcaggcattg agtgcccaag agggatttga gagaataaaa 3360
   caaaagtatg ctgagttgaa ggaccggttg ggtcagagtt ccatgctggg tgagcagggt 3420
   gcccggatcc agagtgtgaa gacagaggca gaggagctgt ttggggagac catggagatg 3480
   atggacagga tgaaagacat ggagttggag ctgctgcggg gcagccaggc catcatgctg 3540
   cgctcagcgg acctgacagg actggagaag cgtgtggagc agatccgtga ccacatcaat 3600
   gggcgcgtgc tctactatgc cacctgcaag tgatgctaca cgttccagcc cgttgcccca 3660
   ctcatctgcg cgctttgctt ttggttgggg ggcagattgg gttggaatgc tttccatctc 3720
   caggagactt tcatgtagcc caaagtacag cctggaccac ccctggtgtg tgtagctagt 3780
   aagattaccc tgagctgcag ctgagcctga gccaatggga cagttacact tgacagacaa 3840
   agatggtgga gattggcatg ccattgaaac taagagctct caagtcaagg aagctgggct 3900
   gggcagtatc cccgcctttt agttctccac 3930
<210> 4
   <211> 1170
   <212> PRT
   <213> Human
<400> 4
<210> 5
   <211> 5200
   <212> DNA
   <213> Human
<220>
   <221> CDS
   <222> (118)...(3696)
<300>
   <301> Kallunki,P., Sainio, K., Eddy, R., Byers, M., Kallunki, T., Sariola,H., Beck, K., Hirvonen, H., Shows, T. B. and Tryggvason, K. <302> A truncated laminin chain homologous to the B2 chain: structure, spatial expression, and chromosomal assignment <303> J. Cell Biol. <304> 119 <306> 679-693 <307> 1992
<400> 5
   gaccacctga tcgaaggaaa aggaaggcac agcggagcgc agagtgagaa ccaccaaccg 60
   aggcgccggg cagcgacccc tgcagcggag acagagactg agcggcccgg caccgccatg 120
   cctgcgctct ggctgggctg ctgcctctgc ttctcgctcc tcctgcccgc agcccgggcc 180
   acctccagga gggaagtctg tgattgcaat gggaagtcca ggcagtgtat ctttgatcgg 240
   gaacttcaca gacaaactgg taatggattc cgctgcctca actgcaatga caacactgat 300
   ggcattcact gcgagaagtg caagaatggc ttttaccggc acagagaaag ggaccgctgt 360
   ttgccctgca attgtaactc caaaggttct cttagtgctc gatgtgacaa ctctggacgg 420
   tgcagctgta aaccaggtgt gacaggagcc agatgcgacc gatgtctgcc aggcttccac 480
   atgctcacgg atgcggggtg cacccaagac cagagactgc tagactccaa gtgtgactgt 540
   gacccagctg gcatcgcagg gccctgtgac gcgggccgct gtgtctgcaa gccagctgtt 600
   actggagaac gctgtgatag gtgtcgatca ggttactata atctggatgg ggggaaccct 660
   gagggctgta cccagtgttt ctgctatggg cattcagcca gctgccgcag ctctgcagaa 720
   tacagtgtcc ataagatcac ctctaccttt catcaagatg ttgatggctg gaaggctgtc 780
   caacgaaatg ggtctcctgc aaagctccaa tggtcacagc gccatcaaga tgtgtttagc 840
   tcagcccaac gactagatcc tgtctatttt gtggctcctg ccaaatttct tgggaatcaa 900
   caggtgagct atgggcaaag cctgtccttt gactaccgtg tggacagagg aggcagacac 960
   ccatctgccc atgatgtgat cctggaaggt gctggtctac ggatcacagc tcccttgatg 1020
   ccacttggca agacactgcc ttgtgggctc accaagactt acacattcag gttaaatgag 1080
   catccaagca ataattggag cccccagctg agttactttg agtatcgaag gttactgcgg 1140
   aatctcacag ccctccgcat ccgagctaca tatggagaat acagtactgg gtacattgac 1200
   aatgtgaccc tgatttcagc ccgccctgtc tctggagccc cagcaccctg ggttgaacag 1260
   tgtatatgtc ctgttgggta caaggggcaa ttctgccagg attgtgcttc tggctacaag 1320
   agagattcag cgagactggg gccttttggc acctgtattc cttgtaactg tcaaggggga 1380
   ggggcctgtg atccagacac aggagattgt tattcagggg atgagaatcc tgacattgag 1440
   tgtgctgact gcccaattgg tttctacaac gatccgcacg acccccgcag ctgcaagcca 1500
   tgtccctgtc ataacgggtt cagctgctca gtgattccgg agacggagga ggtggtgtgc 1560
   aataactgcc ctcccggggt caccggtgcc cgctgtgagc tctgtgctga tggctacttt 1620
   ggggacccct ttggtgaaca tggcccagtg aggccttgtc agccctgtca atgcaacagc 1680
   aatgtggacc ccagtgcctc tgggaattgt gaccggctga caggcaggtg tttgaagtgt 1740
   atccacaaca cagccggcat ctactgcgac cagtgcaaag caggctactt cggggaccca 1800
   ttggctccca acccagcaga caagtgtcga gcttgcaact gtaaccccat gggctcagag 1860
   cctgtaggat gtcgaagtga tggcacctgt gtttgcaagc caggatttgg tggccccaac 1920
   tgtgagcatg gagcattcag ctgtccagct tgctataatc aagtgaagat tcagatggat 1980
   cagtttatgc agcagcttca gagaatggag gccctgattt caaaggctca gggtggtgat 2040
   ggagtagtac ctgatacaga gctggaaggc aggatgcagc aggctgagca ggcccttcag 2100
   gacattctga gagatgccca gatttcagaa ggtgctagca gatcccttgg tctccagttg 2160
   gccaaggtga ggagccaaga gaacagctac cagagccgcc tggatgacct caagatgact 2220
   gtggaaagag ttcgggctct gggaagtcag taccagaacc gagttcggga tactcacagg 2280
   ctcatcactc agatgcagct gagcctggca gaaagtgaag cttccttggg aaacactaac 2340
   attcctgcct cagaccacta cgtggggcca aatggcttta aaagtctggc tcaggaggcc 2400
   acaagattag cagaaagcca cgttgagtca gccagtaaca tggagcaact gacaagggaa 2460
   actgaggact attccaaaca agccctctca ctggtgcgca aggccctgca tgaaggagtc 2520
   ggaagcggaa gcggtagccc ggacggtgct gtggtgcaag ggcttgtgga aaaattggag 2580
   aaaaccaagt ccctggccca gcagttgaca agggaggcca ctcaagcgga aattgaagca 2640
   gataggtctt atcagcacag tctccgcctc ctggattcag tgtctccgct tcagggagtc 2700
   agtgatcagt cctttcaggt ggaagaagca aagaggatca aacaaaaagc ggattcactc 2760
   tcaagcctgg taaccaggca tatggatgag ttcaagcgta cacaaaagaa tctgggaaac 2820
   tggaaagaag aagcacagca gctcttacag aatggaaaaa gtgggagaga gaaatcagat 2880
   cagctgcttt cccgtgccaa tcttgctaaa agcagagcac aagaagcact gagtatgggc 2940
   aatgccactt tttatgaagt tgagagcatc cttaaaaacc tcagagagtt tgacctgcag 3000
   gtggacaaca gaaaagcaga agctgaagaa gccatgaaga gactctccta catcagccag 3060
   aaggtttcag atgccagtga caagacccag caagcagaaa gagccctggg gagcgctgct 3120
   gctgatgcac agagggcaaa gaatggggcc ggggaggccc tggaaatctc cagtgagatt 3180
   gaacaggaga ttgggagtct gaacttggaa gccaatgtga cagcagatgg agccttggcc 3240
   atggaaaagg gactggcctc tctgaagagt gagatgaggg aagtggaagg agagctggaa 3300
   aggaaggagc tggagtttga cacgaatatg gatgcagtac agatggtgat tacagaagcc 3360
   cagaaggttg ataccagagc caagaacgct ggggttacaa tccaagacac actcaacaca 3420
   ttagacggcc tcctgcatct gatggaccag cctctcagtg tagatgaaga ggggctggtc 3480
   ttactggagc agaagctttc ccgagccaag acccagatca acagccaact gcggcccatg 3540
   atgtcagagc tggaagagag ggcacgtcag cagaggggcc acctccattt gctggagaca 3600
   agcatagatg ggattctggc tgatgtgaag aacttggaga acattaggga caacctgccc 3660
   ccaggctgct acaataccca ggctcttgag caacagtgaa gctgccataa atatttctca 3720
   actgaggttc ttgggataca gatctcaggg ctcgggagcc atgtcatgtg agtgggtggg 3780
   atggggacat ttgaacatgt ttaatgggta tgctcaggtc aactgacctg accccattcc 3840
   tgatcccatg gccaggtggt tgtcttattg caccatactc cttgcttcct gatgctgggc 3900
   atgaggcaga taggcactgg tgtgagaatg atcaaggatc tggaccccaa agatagactg 3960
   gatggaaaga caaactgcac aggcagatgt ttgcctcata atagtcgtaa gtggagtcct 4020
   ggaatttgga caagtgctgt tgggatatag tcaacttatt ctttgagtaa tgtgactaaa 4080
   ggaaaaaact ttgactttgc ccaggcatga aattcttcct aatgtcagaa cagagtgcaa 4140
   cccagtcaca ctgtggccag taaaatacta ttgcctcata ttgtcctctg caagcttctt 4200
   gctgatcaga gttcctccta cttacaaccc agggtgtgaa catgttctcc attttcaagc 4260
   tggaagaagt gagcagtgtt ggagtgagga cctgtaaggc aggcccattc agagctatgg 4320
   tgcttgctgg tgcctgccac cttcaagttc tggacctggg catgacatcc tttcttttaa 4380
   tgatgccatg gcaacttaga gattgcattt ttattaaagc atttcctacc agcaaagcaa 4440
   atgttgggaa agtatttact ttttcggttt caaagtgata gaaaagtgtg gcttgggcat 4500
   tgaaagaggt aaaattctct agatttatta gtcctaattc aatcctactt ttcgaacacc 4560
   aaaaatgatg cgcatcaatg tattttatct tattttctca atctcctctc tctttcctcc 4620
   acccataata agagaatgtt cctactcaca cttcagctgg gtcacatcca tccctccatt 4680
   catccttcca tccatctttc catccattac ctccatccat ccttccaaca tatatttatt 4740
   gagtacctac tgtgtgccag gggctggtgg gacagtggtg acatagtctc tgccctcata 4800
   gagttgattg tctagtgagg aagacaagca tttttaaaaa ataaatttaa acttacaaac 4860
   tttgtttgtc acaagtggtg tttattgcaa taaccgcttg gtttgcaacc tctttgctca 4920
   acagaacata tgttgcaaga ccctcccatg ggcactgagt ttggcaagga tgacagagct 4980
   ctgggttgtg cacatttctt tgcattccag cgtcactctg tgccttctac aactgattgc 5040
   aacagactgt tgagttatga taacaccagt gggaattgct ggaggaacca gaggcacttc 5100
   caccttggct gggaagacta tggtgctgcc ttgcttctgt atttccttgg attttcctga 5160
   aagtgttttt aaataaagaa caattgttag atgccaaaaa 5200
<210> 6
   <211> 1193
   <212> PRT
   <213> Human
<400> 6
<210> 7
   <211> 28
   <212> DNA
   <213> Artificial Sequence
<210>
   <223> primer for PCR amplification
<400> 7
   tgtggggccc tgaaaggcga gctgagat 28
<210> 8
   <211> 28
   <212> DNA
   <213> Artificial Sequence
<220>
   <223> primer for PCR amplification
<400> 8
   atgggccgcc atacgacgac gctcaact 28
<210> 9
   <211> 24
   <212> DNA
   <213> Artificial Sequence
<220>
   <223> primer for PCR amplification
<400> 9
   actgcccctc atcagactgc tact 24
<210> 10
   <211> 24
   <212> DNA
   <213> Artificial Sequence
<220>
   <223> primer for PCR amplification
<400> 10
   cactgccttg tactcgggta gctg 24
<210> 11
   <211> 20
   <212> DNA
   <213> Artificial Sequence
<220>
   <223> primer for PCR amplification
<400> 11
   aagcagaaga tgcggactgt 20
<210> 12
   <211> 20
   <212> DNA
   <213> Artificial Sequence
<220>
   <223> primer for PCR amplification
<400> 12
   accactggtt tttctgccac 20
<210> 13
   <211> 23
   <212> DNA
   <213> Artificial Sequence
<220>
   <223> primer for PCR amplification
<400> 13
   tctttccacc aggcccccgg ctc 23
<210> 14
   <211> 23
   <212> DNA
   <213> Artificial Sequence
<220>
   <223> primer for PCR amplification
<400> 14
   tgcgggcgga catggggaga tcc 23
<210> 15
   <211> 24
   <212> DNA
   <213> Artificial Sequence
<220>
   <223> primer for PCR amplification
<400> 15
   acgagtggca gtttcttctt ggga 24
<210> 16
   <211> 24
   <212> DNA
   <213> Artificial Sequence
<220>
   <223> primer for PCR amplification
<400> 16
   tatgactcac ttccaggggg cact 24
<210> 17
   <211> 23
   <212> DNA
   <213> Artificial Sequence
<220>
   <223> primer for PCR amplification
<400> 17
   tagagctaga ctccgggcga tga 23
<210> 18
   <211> 23
   <212> DNA
   <213> Artificial Sequence
<220>
   <223> primer for PCR amplification
<400> 18
   ttgccttaaa caagaccacg aaa 23
<210> 19
   <211> 23
   <212> DNA
   <213> Artificial Sequence
<220>
   <223> primer for PCR amplification
<400> 19
   ggatgtcccg gtgactacgt ctg 23
<210> 20
   <211> 23
   <212> DNA
   <213> Artificial Sequence
<220>
   <223> primer for PCR amplification
<400> 20
   ggcggatctg gttatcgaag ggt 23
<210> 21
   <211> 20
   <212> DNA
   <213> Artificial Sequence
<220>
   <223> primer for PCR amplification
<400> 21
   caccatggag aaggccgggg 20
<210> 22
   <211> 20
   <212> DNA
   <213> Artificial Sequence
<220>
   <223> primer for PCR amplification
<400> 22
   gacggacaca ttgggggtag 20
<210> 23
   <211> 24
   <212> DNA
   <213> Artificial Sequence
<220>
   <223> primer for PCR amplification
<400> 23
   gacaggggga ggggaggagc tagg 24
<210> 24
   <211> 26
   <212> DNA
   <213> Artificial Sequence
<220>
   <223> primer for PCR amplification
<400> 24
   cttccctcca accagttgcc ccaaac 26
<210> 25
   <211> 25
   <212> DNA
   <213> Artificial Sequence
<220>
   <223> primer for PCR amplification
<400> 25
   cagccctgat tcttccacca gtccc 25
<210> 26
   <211> 25
   <212> DNA
   <213> Artificial Sequence
<220>
   <223> primer for PCR amplification
<400> 26
   tggaaggttc ccagtcgggt tcacc 25
<210> 27
   <211> 26
   <212> DNA
   <213> Artificial Sequence
<220>
   <223> primer for PCR amplification
<400> 27
   gggaaatggg aggggtgcaa aagagg 26
<210> 28
   <211> 26
   <212> DNA
   <213> Artificial Sequence
<220>
   <223> primer for PCR amplification
<400> 28
   ttgcgtgagt gtggatggga ttggtg 26
<210> 29
   <211> 20
   <212> DNA
   <213> Artificial Sequence
<220>
   <223> primer for PCR amplification
<400> 29
   gtggacctga cctgccgtct 20
<210> 30
   <211> 20
   <212> DNA
   <213> Artificial Sequence
<220>
   <223> primer for PCR amplification
<400> 30
   ggaggagtgg gtgtcgctgt 20
*<*210> 31
   <211> 26
   <212> DNA
   <213> Artificial Sequence
<220>
   <223> primer for PCR amplification
<400> 31
   cgctttcatg gtgtgggcta aggacg 26
<210> 32
   <211> 26
   <212> DNA
   <213> Artificial Sequence
<220>
   <223> primer for PCR amplification
<400> 32
   tagttggggt ggtcctgcat gtgctg 26
<210> 33
   <211> 28
   <212> DNA
   <213> Artificial Sequence
<220>
   <223> primer for PCR amplification
<400> 33
   gaatgctgca aactgaccac gctggaac 28
<210> 34
   <211> 28
   <212> DNA
   <213> Artificial Sequence
<220>
   <223> primer for PCR amplification
<400> 34
   tggcattcaa gagggttttc agtctgga 28
<210> 35
   <211> 26
   <212> DNA
   <213> Artificial Sequence
<220>
   <223> primer for PCR amplification
<400> 35
   gccctctccc tcccctccac gcacag 26
<210> 36
   <211> 26
   <212> DNA
   <213> Artificial Sequence
<220>
   <223> primer for PCR amplification
<400> 36
   cggcgccgtt gctcacagac cacagg 26
<210> 37
   <211> 24
   <212> DNA
   <213> Artificial Sequence
<220>
   <223> primer for PCR amplification
<400> 37
   cgagaggacc ccgtggatgc agag 24
<210> 38
   <211> 24
   <212> DNA
   <213> Artificial Sequence
<220>
   <223> primer for PCR amplification
<400> 38
   ggcggccatc ttcagcttct ccag 24
<210> 39
   <211> 28
   <212> DNA
   <213> Artificial Sequence
<220>
   <223> primer for PCR amplification
<400> 39
   acccattatc cagatgtgtt tgcccgag 28
<210> 40
   <211> 26
   <212> DNA
   <213> Artificial Sequence
<220>
   <223> primer for PCR amplification
<400> 40
   atggtgaagc tgggcatagg cggcag 26
<210> 41
   <211> 20
   <212> DNA
   <213> Artificial Sequence
<220>
   <223> primer for PCR amplification
<400> 41
   gcagagcaaa ggagaggaaa 20
<210> 42
   <211> 20
   <212> DNA
   <213> Artificial Sequence
<220>
   <223> primer for PCR amplification
<400> 42
   cagggacaga gccagacact 20

## Description

### TECHNICAL FIELD

The present invention relates to a method for proliferation of pluripotent stem cells, the use of laminin-5 as a cell-supporting material, and a culture kit for pluripotent stem cells.

The present application claims priority based on Japanese Patent Application Nos. 2008-93350 (filed on March 31, 2008) and 2008-225686 (filed on September 3, 2008).

### BACKGROUND ART

### Method for proliferation of pluripotent stem cells

Pluripotent stem cells are stem cells having the ability to differentiate into cells of every tissue type (differentiation pluripotency). For the sake of information, cells currently known as pluripotent stem cells include embryonic stem cells (ES cells), induced pluripotent stem cells (iPS cells) which are prepared from somatic cells by introducing and expressing a combination of specific factors (e.g., a combination of Oct3/4, Sox2, Klf4 and c-Myc), embryonic germ cells (EG cells) which are prepared from primordial germ cells, and germline stem cells (GS cells) which are prepared from germ cells in the testis.

For information only, embryonic stem cells (ES cells) are pluripotent stem cells established from the inner cell mass (ICM) of blastocysts at the early developmental stage (Nature. 292, 154-156, 1981, Proc. Natl. Acad. Sci. USA. 78, 7634-7638, 1981, Science. 282, 1145-1147, 1998). Mouse ES cells can retain their pluripotency in the presence of leukemia inhibitory factor (LIF) (Nature. 336, 684-687, 1988, Nature. 336, 688-690, 1988). For their maintenance culture, in general, LIF-producing cell lines or mouse embryonic fibroblasts (MEFs) are used as feeder cells, or alternatively, a LIF-supplemented medium and an appropriate supporting material are used instead.

In the culture of mouse ES cells, feeder cells are responsible for providing a scaffold for cell adhesion and supplying growth factors required for ES cells and LIF. It should be noted that the culture solution may further be supplemented with LIF, in addition to LIF supplied from feeder cells. In embodiments where LIF is added, LIF per se or the supernatant of a LIF-producing cell line may be added to the culture solution. On the other hand, in the case of feeder cell-free systems, mouse ES cells are cultured in a culture solution supplemented with LIF by using various extracellular matrixes (e.g., gelatin) as supporting materials.

In contrast, the culture of human ES cells requires the presence of basic fibroblast growth factor (FGF2) to maintain their pluripotency (Dev Biol. 227, 271-278, 2000), and also requires additional factors supplied from MEFs for this purpose. Human ES cells can be maintained and cultured in a state retaining their pluripotency, either by using MEFs as feeder cells in the presence of FGF2 or by using the supernatant of MEFs in combination with an appropriate supporting material (Nature Biotech. 19, 971-974, 2001).

More specifically, human ES cells may be cultured in the presence of serum in addition to FGF2 or may be cultured in a serum-free medium. Currently, it is more common to use a serum-free medium for culture of human ES cells, and serum replacements used for this purpose include Knockout^{™} serum replacement (KSR, Invitrogen) and so on. Culture systems commonly used for human ES cells include those in which MEFs are used as feeder cells and the culture solution is supplemented with FGF2, or those in which various extracellular matrix proteins (e.g., Matrigel) are used as supporting materials and the cells are cultured in the supernatant of MEFs supplemented with FGF2. It should be noted that not only mouse fibroblasts, but also human fibroblasts can be used as feeder cells.

Further, recent reports have shown that induced pluripotent stem cells (iPS cells) having properties very similar to those of embryonic stem cells were established from somatic cells in mice and humans (Cell. 126, 663-672, 2006, Cell. 131, 861-872, 2007, Science. 318, 1917-1920, 2007, WO2007/069666). iPS cells are somatic cell-derived pluripotent stem cells established by introducing Oct3/4, Sox2, Klf4, c-Myc and/or other factors into somatic cells. In general, the presence of feeder cells is also required for maintenance culture of iPS cells, as in the case of ES cells.

iPS cells can be cultured in the same manner as used for ES cells. Mouse iPS cells can be maintained and cultured on STO cells (cell line derived from mouse SIM fibroblasts stably producing LIF) using the supernatant of LIF-producing cells. On the other hand, in feeder cell-free systems, mouse iPS cells can be maintained and cultured on gelatin-coated plates when LIF is added to their medium. It should be noted that STO cells are known to be effective in stably maintaining mouse-derived ES cells, EG cells and EC cells.

Human iPS cells can also be cultured in FGF2-supplemented systems by using mouse fibroblasts as feeder cells. It should be noted that the group of Shinya Yamanaka et al. (Kyoto University) uses SNL cells (cell line derived from mouse fibroblasts co-expressing LIF and the G418 resistance gene) for culture of human iPS cells (Cell. 131, 861-872, 2007). In the case of using feeder cell-free systems for maintenance culture of human iPS cells, Matrigel is used as a supporting material and FGF2 is added to the supernatant of MEFs.

Somatic cell-derived iPS cells have fewer ethical problems than early embryo-derived embryonic stem cells, and are free from the problem of immunological rejection because they can be prepared from patients' own cells. Their application to regenerative medicine is expected.

For the sake of a complete disclosure and information, embryonic germ cells (EG cells) are cells established from primordial germ cells by being cultured in the presence of Steel Factor (Kit-Ligand), LIF and FGF2, and are known to have substantially the same properties as ES cells, as demonstrated by experiments in mice. For maintenance culture of EG cells, it is possible to use the same culture method as used for ES cells. Namely, EG cells can be cultured in LIF-supplemented systems in the presence of feeder cells such as MEFs or STO cells (Cell 70:841-847, 1992, Development 120, 3197-3120, 1994)..

Germline stem cells (GS cells) prepared from germ cells in the testis are cell lines of spermatogonial stem cells (sperm stem cells) designed to allow *in vitro* culture under culture conditions containing at least GDNF (Glial cell-line derived neurotrophic growth factor), and they can form sperms when injected into seminiferous tubules in the testis. Prolonged culture of GS cells can be accomplished on MEFs (as feeder cells) by using a medium supplemented with GDNF, FGF2, EGF (epidermal growth factor) and LIF. It has also been reported that GS cells were cultured in feeder cell-free systems; GS cells can be maintained by being cultured on laminin-coated plates (Biology of Reproduction 69:612-616, 2003, Biology of Reproduction 72:985-991, 2005).

For information only: Among GS cells, mGS cells (multipotent germline stem cells) particularly have the same properties as ES cells and also have differentiation pluripotency. mGS cells are established by further converting the established GS cells into pluripotent stem cells in culture systems for ES cells. The established mGS cells can also be cultured in the same manner as used for ES cells, i.e., can be cultured in systems using a LIF-supplemented medium in the presence of feeder cells (Cell 119:1001-1012, 2004, Nature 440:1199-1203, 2006).

Such pluripotent cells as described above are expected to have applications to regenerative medicine, etc. However, particularly for their clinical applications, it is necessary to avoid the problem of immunological rejection and potential risks such as contamination with unknown viruses; and hence there is a need to develop a maintenance culture system in which no animal-derived substance is used whatsoever. Namely, it is predicted that when animal-derived sialic acids, which cannot be found in humans, are taken up into human ES cells or iPS cells, the antigenicity of these sialic acids will become a problem and will cause immunological rejection in the regenerated tissue. Moreover, even in the case of using human proteins, naturally occurring proteins prepared from placenta and other tissues may have potential risks such as contamination with AIDS virus (HIV), hepatitis C virus (HCV) and other unknown viruses.

For these reasons, while maintaining the pluripotency of human ES cells, iPS cells and other pluripotent stem cells, efforts have been made to search for the composition of culture solution capable of supporting their proliferation and/or appropriate supporting materials serving as substitutes for MEFs (Nature Biotech. 24, 185-187, 2006, Stem Cell. 24, 2649-2660, 2006). However, even when MEFs are used as feeder cells, the cell adhesion efficiency obtained is as low as a few percent, and this efficiency further decreases in feeder cell-free systems currently under study. In the maintenance culture of pluripotent stem cells, it is desired to develop a system in which no animal-derived substance is used whatsoever, although low cell adhesion efficiency as described above is one of the great problems. Thus, there is a demand for human-derived supporting materials, which exert effective adhesion activity while maintaining pluripotency, and such supporting materials may serve as useful tools to obtain cellular materials that can be used in clinical applications such as regenerative medicine.

### Laminin-5

Laminin, which is localized primarily on the basement membranes of various tissues, is an extracellular matrix protein playing an important role in maintenance of tissue structure and in control of cell functions (Matrix Biol., 18:19-28, 1999, Dev. Dyn., 218:213-234, 2000).

The structure of laminin is a heterotrimer molecule composed of α, β and γ chains linked to each other via disulfide linkages, which takes a characteristic cross-structure. Each chain is composed of multiple domains, and domains I and II form a triple helix. Before the filing date of the present application, at least 15 isoforms of laminin molecules have been identified according to different combinations of 5 types of α chains (α1 to α5), 3 types of β chains (β1 to β3) and 3 types of γ chains (γ1 to γ3), and it is suggested that there are actually several times that number of isoforms (Miyazaki et al., Jikken Igaku (Experimental Medicine) Vol. 16 No. 16 (extra issue), 1998, pages 114-119, Dev. Dyn., 218:213-234, 2000, J. Neurosci., 20:6517-6528, 2000, Physiol Rev. 85, 979-1000, 2005). These α, β and γ chains are encoded by different genes, respectively; and the individual laminin isoforms have specific sites of localization and specific functions, and mainly regulate cell adhesion, proliferation, motility, differentiation and so on through the cell membrane receptor integrin (Dev. Dyn. 218, 213-234, 2000, Physiol. Rev. 85, 979-1000, 2005).

Table 1 shows 15 laminin molecular species and their subunit structure.

**[Table 1]**

| Laminin molecular species and subunit structure | | |
|---|---|---|
| Name | Structure | Also called |
| Laminin-1 | α1β1γ1 | EHS laminin |
| Laminin-2 | α2β1γ1 | Merosin |
| Laminin-3 | α1β2γ1 | S-Laminin |
| Laminin-4 | α2β2γ1 | S-Merosin |
| Laminin-5 | α3β3γ2 | Ladsin/epiligrin/ kalinin/nicein |
| Laminin-6 | α3β1γ1 | K-Laminin |
| Laminin-7 | α3β2γ1 | KS-Laminin |
| Laminin-8 | α4β1γ1 | |
| Laminin-9 | α4β2γ1 | |
| Laminin-10 | α5β1γ1 | |
| Laminin-11 | α5β2γ1 | |
| Laminin-12 | α2β1γ3 | |
| Laminin-13 | α3β2γ3 | |
| Laminin-14 | α4β2γ3 | |
| Laminin-15 | α5β2γ3 | |

Laminin molecules construct the basement membrane by associating with each other at the amino (N) terminal portion (short arm) of the triple strand or by associating with other matrix molecules. On the other hand, laminin molecules each have 5 homologous globular domains (G1-G5 domains or LG1-LG5) at the carboxy (C) terminal of the α chain, and bind to integrin or other receptors mainly at this site.

Laminin-5 (also called kalinin, epiligrin, nicein or ladsin) is one of the laminin isoforms, which is composed of α3, β3 and γ2 chains, and was found by multiple research institutes under different circumstances (J. Cell Biol. 114, 567-576,1991, Cell 65, 599-610, 1991, J. Invest Dermatol. 101, 738-743, 1993, Proc. Natl. Acad. Sci. USA. 90, 11767-11771, 1993).

Laminin-5 is reported to have strong cell adhesion activity, cell dispersion activity, cell proliferation activity and the like on various cells (Proc. Natl. Acad. Sci. USA. 90, 11767-11771, 1993, J. Biochem. 116, 862-869, 1994, J. Cell Biol. 125, 205-214, 1994, Mol. Biol. Cell. 16, 881-890, 2005, Stem Cell. 24, 2346-2354, 2006). WO2007/023875 discloses culture techniques for mesenchymal stem cells using laminin-5.

### CITATION LIST

### PATENT LITERATURE

[PTL 1] WO2007/069666
[PTL 2] WO2007/023875
[PTL 3] JP 2001-172196 A

### NON PATENT LITERATURE

[NPL 1] Nature. 292, 154-156, 1981
[NPL2] Proc. Natl. Acad. Sci. USA. 78, 7634-7638,1981
[NPL 3] Science. 282, 1145-1147, 1998
[NPL 4] Nature. 336, 684-687, 1988
[NPL 5] Nature. 336, 688-690, 1988
[NPL 6] Dev. Biol. 227, 271-278, 2000
[NPL 7] Nature Biotech. 19, 971-974, 2001
[NPL 8] Cell. 126, 663-672, 2006
[NPL 9] Science. 318, 1917-1920, 2007
[NPL 10] Nature Biotech. 24, 185-187, 2006
[NPL 11] Stem Cell. 24, 2649-2660, 2006
[NPL 12] Matrix Biol., 18:19-28, 1999
[NPL 13] Dev. Dyn., 218:213-234, 2000
[NPL 14] Miyazaki et al., Jikken Igaku (Experimental Medicine) Vol. 16, No. 16 (extra issue), 114-119, 1998
[NPL 15] J. Neurosci., 20:6517-6528, 2000
[NPL 16] Physiol. Rev. 85, 979-1000, 2005
[NPL 17] J. Cell Biol. 114, 567-576, 1991
[NPL 18] Cell. 65, 599-610, 1991
[NPL 19] J. Invest Dermatol. 101, 738-743, 1993
[NPL 20] Proc. Natl. Acad. Sci. USA. 90, 11767-11771, 1993
[NPL 21] J. Biochem. 116, 862-869, 1994
[NPL 22] J. Cell Biol. 125, 205-214, 1994
[NPL 23] Mol. Biol. Cell. 16, 881-890, 2005
[NPL 24] Stem Cell. 24, 2346-2354, 2006
[NPL 25] Dev. Biol. 163: p. 288-292, 1994
[NPL 26] Dev. Biol. 127: p. 224-227, 1988
[NPL 27] Reprod. Fertil. Dev. 6: p. 563-568, 1994
[NPL 28] Reprod. Fertil. Dev. 6: p. 553-562, 1994
[NPL 29] Proc. Natl. Acad. Sci. USA 92: p. 7844-7848, 1995
[NPL 30] Proc. Natl. Acad. Sci. USA 95:13726-13731, 1998
[NPL 31] Nature Biotech., 18, p. 399-404, 2000
[NPL 32] Nature 439: 216-219, 2006
[NPL 33] Cell Stem Cell 2: 113-117, 2008
[NPL 34] Stem Cells 24: 2669-2676, 2006
[NPL 35] Curr. Biol., 11: p. 1553-1558, 2001
[NPL 36] Nature Biotechnol 26:101-106, 2008
[NPL 37] Cell Stem Cell 2:10-12, 2008
[NPL 38] Cell 131:861-872, 2007
[NPL 39] Takahashi and Yamanaka, Saibo Kogaku (Cell Technology), Vol. 27, No. 3, 252-253, 2008
[NPL 40] J. Biol. Chem., 280 (2005), 14370-14377
[NPL 41] J. Biol. Chem. 269: p. 22779-22787, 1994
[NPL 42] J. Biol. Chem. 269: p. 11073-11080, 1994
[NPL 43] J. Cell. Biol. 119: p. 679-693, 1992
[NPL 44] Nucleic Acids Res. 25:3389-3402, 1997
[NPL 45] J. Mol. Biol. 215:403-410,1990
[NPL 46] J. Mol. Biol. 147:195-197, 1981
[NPL 47] Cell 70:841-847, 1992
[NPL 48] Development 120, 3197-3120, 1994
[NPL 49] Biology of Reproduction 69:612-616, 2003
[NPL 50] Biology of Reproduction 72:985-991, 2005
[NPL 51] Cell 119:1001-1012, 2004
[NPL 52] Nature 440:1199-1203, 2006
[NPL 53] Mol. Reprod. Dev. 36: p. 424-433, 1993
[NPL 54] Nature 454:646-650, 2008
[NPL 55] Cell 136:411-419, 2009
[NPL 56] Cell Stem Cell 3:568-574, 2008
[NPL 57] Science 322:945-949, 2008
[NPL 58] Science 322:949-953, 2008

### SUMMARY OF THE INVENTION

The object of the present invention is to provide a technique for efficient proliferation of pluripotent stem cells in a system free from any animal-derived substance such as feeder cells or serum.

As a result of extensive and intensive efforts made to achieve the above object, the inventors of the present invention have found that the use of laminin-5, an extracellular matrix molecule, allows pluripotent stem cells to proliferate in an undifferentiated state without the need to use feeder cells or serum. This finding led to the completion of the present invention.

Accordingly, to achieve the above object, the present invention provides a method for proliferation of pluripotent stem cells, which comprises culturing the pluripotent stem cells in a medium free from both feeder cells and serum in a system containing laminin-5, wherein the pluripotent stem cells are induced pluripotent cells or embryonic stem cells.

The present invention also provides the use of laminin-5 as a cell-supporting material for a proliferation of induced pluripotent stem cells or embryonic stem cells.

The present invention includes the following embodiments as preferred ones.

### [Embodiment 1]

A method for proliferation of pluripotent stem cells, which comprises culturing the pluripotent stem cells in a medium free from both feeder cells and serum in a system containing laminin-5, wherein the pluripotent stem cells are induced pluripotent cells or embryonic stem cells.

### [Embodiment 2]

The method according to Embodiment 1, wherein the pluripotent stem cells are human induced pluripotent stem cells or human embryonic stem cells.

### [Embodiment 3]

The method according to Embodiment 1 or 2, wherein the culture medium comprises a serum replacement.

### [Embodiment 4]

The method according to Embodiment 6, wherein the serum replacement comprises one or more amino acids selected from the group consisting of glycine, histidine, isoleucine, methionine, phenylalanine, proline, hydroxyproline, serine, threonine, tryptophan, tyrosine and valine, vitamin(s) consisting of thiamine and/or ascorbic acid, one or more trace metal elements selected from the group consisting of silver, aluminum, barium, cadmium, cobalt, chromium, germanium, manganese, silicon, vanadium, molybdenum, nickel, rubidium, tin and zirconium, one or more halogen elements selected from the group consisting of bromine, iodine and fluorine, as well as one or more ingredients selected from the group consisting of albumin, reduced glutathione, transferrin, insulin and sodium selenite.

### [Embodiment 5]

The method according to any one of Embodiments 1 to 4, wherein the induced pluripotent stem cells or embryonic stem cells are cultured in a system containing laminin-5 and (an) additional extracellular matrix protein(s).

### [Embodiment 6]

The method according to Embodiment 5, wherein the additional extracellular matrix protein is collagen.

### [Embodiment 7]

The method according to any one of Embodiments 1 to 6, wherein the induced pluripotent stem cells or embryonic stem cells are cultured in a laminin-5-treated culture vessel.

### [Embodiment 8]

The method according to Embodiment 7, wherein the laminin-5 is human laminin-5.

### [Embodiment 9]

The method according to any one of Embodiments 1 to 8, wherein the pluripotent stem cells do not differentiate during culture.

### [Embodiment 10]

Use of laminin-5 as a cell-supporting material for proliferation of induced pluripotent stem cells or embryonic stem cells.

### ADVANTAGEOUS EFFECTS OF INVENTION

The present invention enables efficient proliferation of pluripotent stem cells while maintaining them in an undifferentiated state, without the need to use feeder cells or serum.

### BRIEF DESCRIPTION OF DRAWINGS

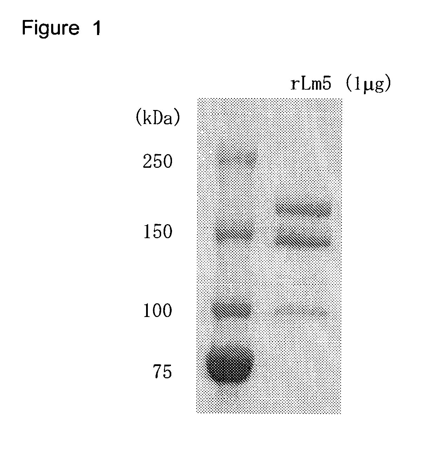

Figure 1 shows electrophoresis of purified recombinant human laminin-5 on an SDS polyacrylamide gel. It should be noted that the right lane in Figure 1 shows the results of electrophoresis of 1 µg recombinant human laminin-5.

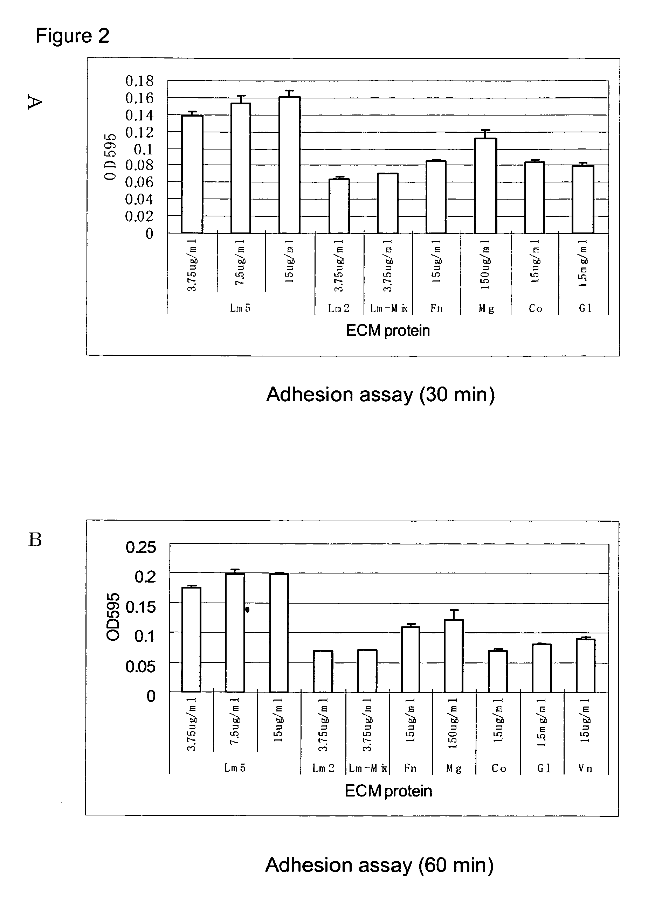

Figure 2 shows a comparison between recombinant human laminin-5 and various extracellular matrix proteins for their effect on the adhesion effect on ES cells. Figure 2A shows the results of adhesion assay after culture for 30 minutes, while Figure 2B shows the results of adhesion assay after culture for 60 minutes.

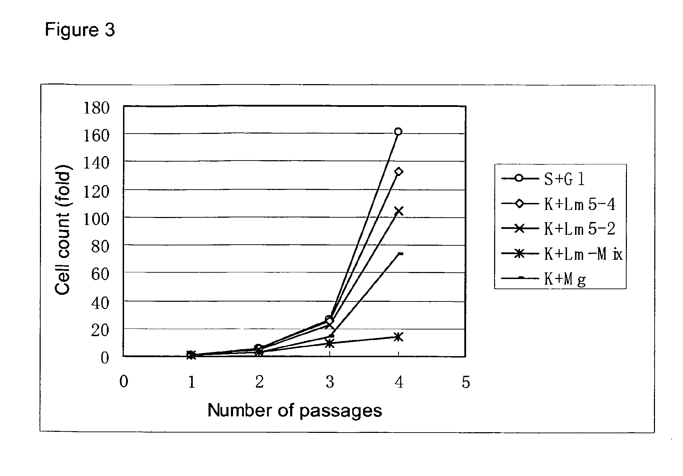

Figure 3 shows a comparison of extracellular matrix proteins for their effect on the proliferation of ES cells in the absence of feeder cells. Figure 3 shows the results of proliferation assay, where open circles represent S+Gl, open diamonds represent K+Lm5-4, crosses represent K+Lm5-2, asterisks represent K+Lm-Mix, and small solid squares represent K+Mg.

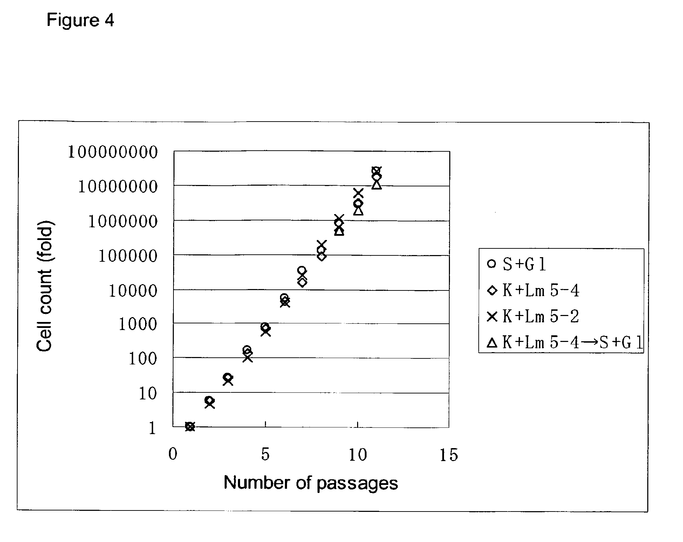

Figure 4 shows a comparison of extracellular matrix proteins for their effect on the prolonged subculture of ES cells in the absence of feeder cells. Figure 4 shows the results of proliferation assay, where open circles represent S+Gl, open diamonds represent K+Lm5-4, crosses represent K+Lm5-2, and open triangles represent K+Lm5-4 → S+G1.

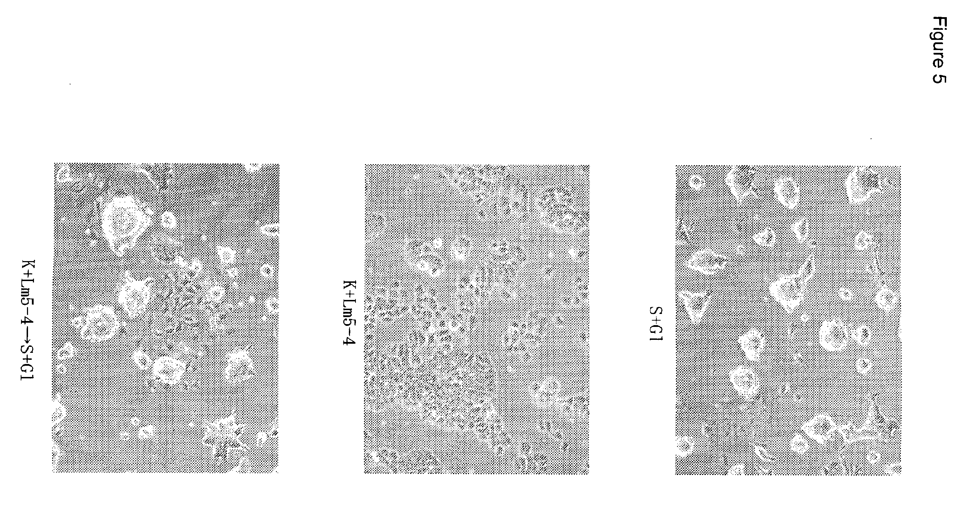

Figure 5 shows the morphology of ES cells when subcultured for a long period of time on recombinant human laminin-5-coated plates in a serum-free medium and when returned to culture on gelatin-coated plates in a serum medium. Figure 5 shows, from the top, the results of S+Gl, K+Lm5-4, and K+Lm5-4 → S+Gl, respectively.

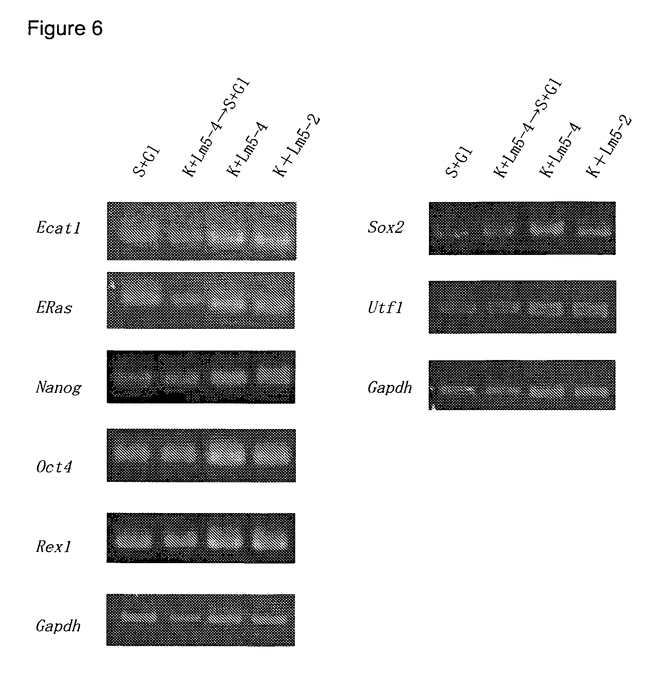

Figure 6 shows the results of RT-PCR compared for the expression of various undifferentiation markers under each culture conditions in ES cells when cultured in a KSR-supplemented medium.

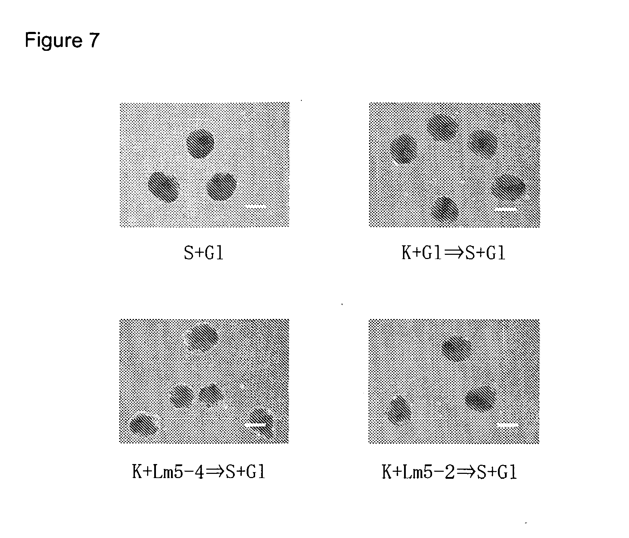

Figure 7 shows embryoid bodies upon culture in a LIF-free maintenance medium, observed for ES cells cultured in a KSR-supplemented medium. In Figure 7, the scale bar represents 250 µm.

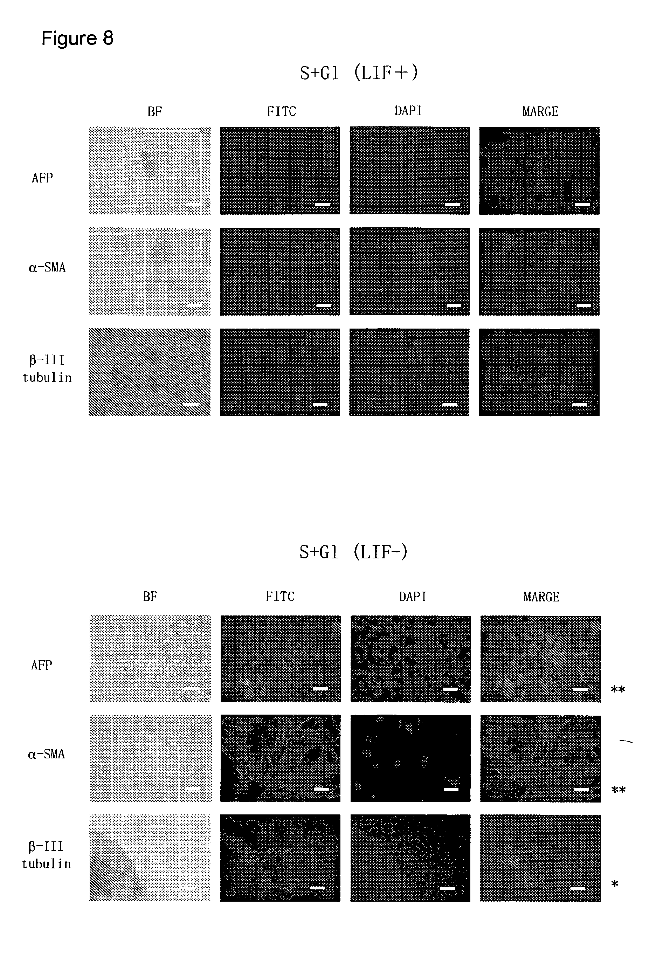

Figure 8 shows the results studied for the differentiation potency of ES cells in a culture system of serum medium on gelatin-coated plates (S+Gl) (Example 5). In Figure 8, the upper panel shows the results of S+Gl (LIF+), while the lower panel shows the results of S+Gl (LIF-). In Figure 8, although the scale bar represents 100 µm, a single asterisk (*) indicates that the scale bar represents 50 µm, and a double asterisk (**) indicates that the scale bar represents 25 µm.

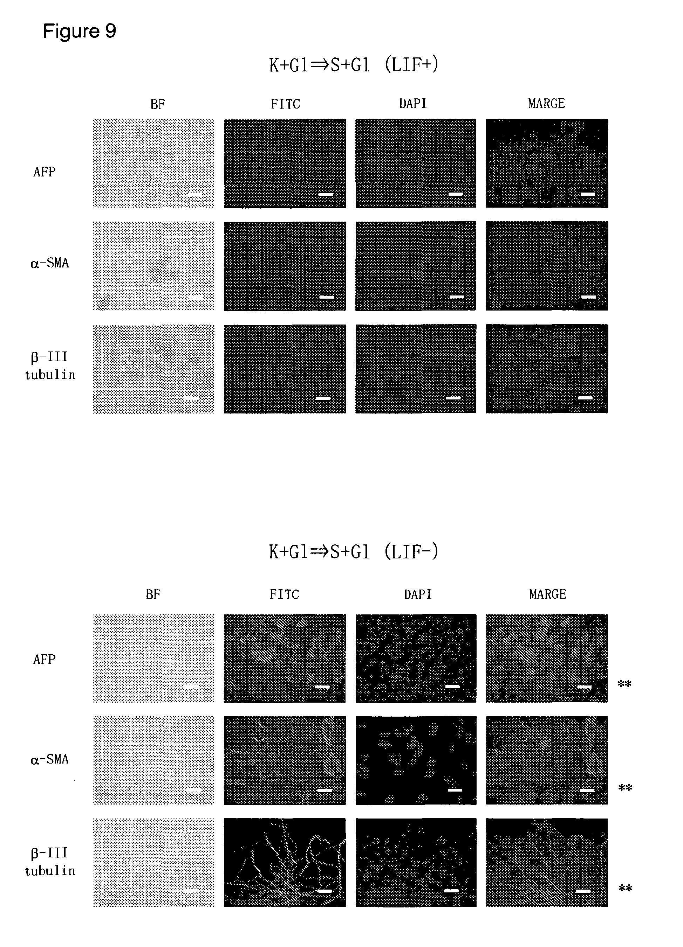

Figure 9 shows the results studied for the differentiation potency of ES cells when cultured in a culture system of KSR-supplemented serum-free medium on gelatin-coated plates (K+Gl) and then returned to a culture system of serum medium (S+Gl) (Example 5). In Figure 9, the upper panel shows the results of K+Gl ⇒ S+Gl (LIF+), while the lower panel shows the results of K+Gl ⇒ S+Gl (LIF-). In Figure 9, although the scale bar represents 100 µm, a double asterisk (**) indicates that the scale bar represents 25 µm.

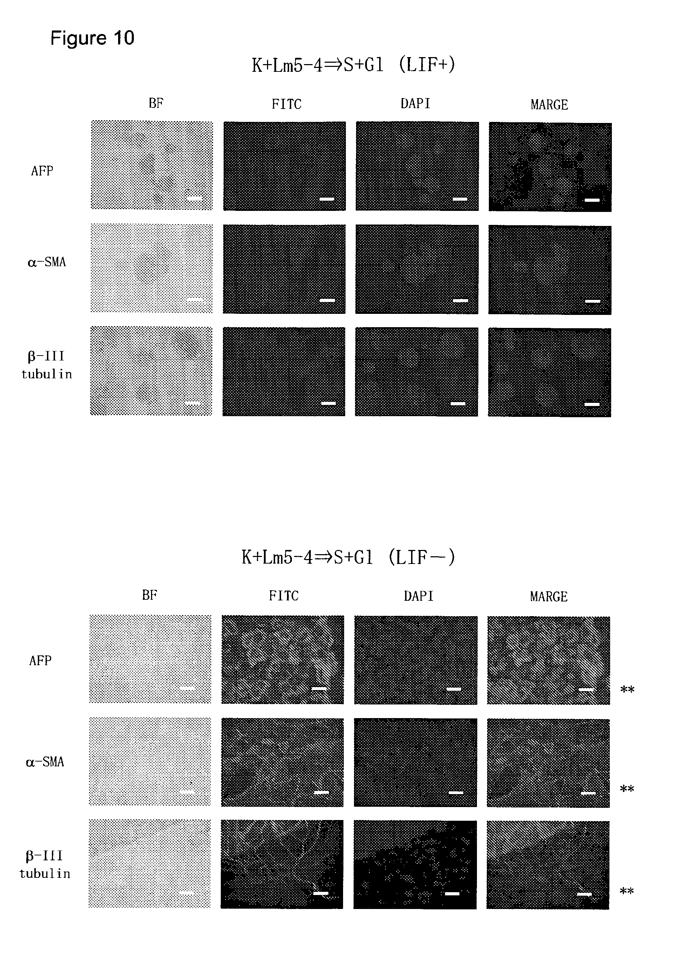

Figure 10 shows the results studied for the differentiation potency of ES cells when cultured in a culture system of KSR-supplemented serum-free medium on 4 µg/ml recombinant human laminin-5-coated plates (K+Lm5-4) and then returned to a culture system of serum medium (S+Gl). In Figure 10, the upper panel shows the results of K+Lm5-4 ⇒ S+Gl (LIF+), while the lower panel shows the results of K+Lm5-4 ⇒ S+Gl (LIF-). In Figure 10, although the scale bar represents 100 µm, a double asterisk (**) indicates that the scale bar represents 25 µm.

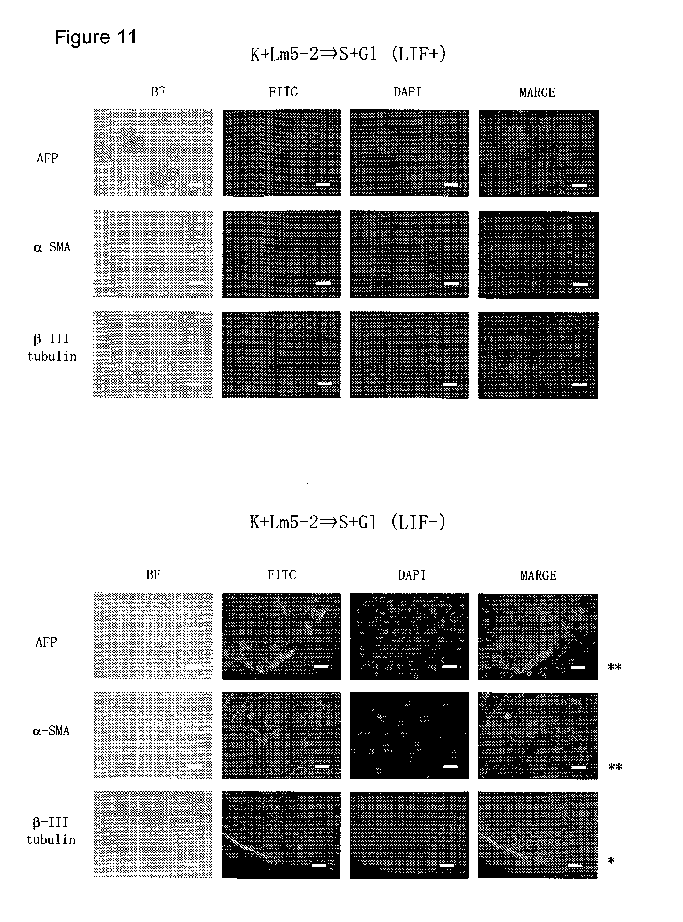

Figure 11 shows the results studied for the differentiation potency of ES cells when cultured in a culture system of KSR-supplemented serum-free medium on 2 µg/ml recombinant human laminin-5-coated plates (K+Lm5-2) and then returned to a culture system of serum medium (S+Gl). In Figure 11, the upper panel shows the results of K+Lm5-2 ⇒ S+Gl (LIF+), while the lower panel shows the results of K+Lm5-2 ⇒ S+Gl (LIF-). In Figure 11, although the scale bar represents 100 µm, a single asterisk (*) indicates that the scale bar represents 50 µm, and a double asterisk (**) indicates that the scale bar represents 25 µm.

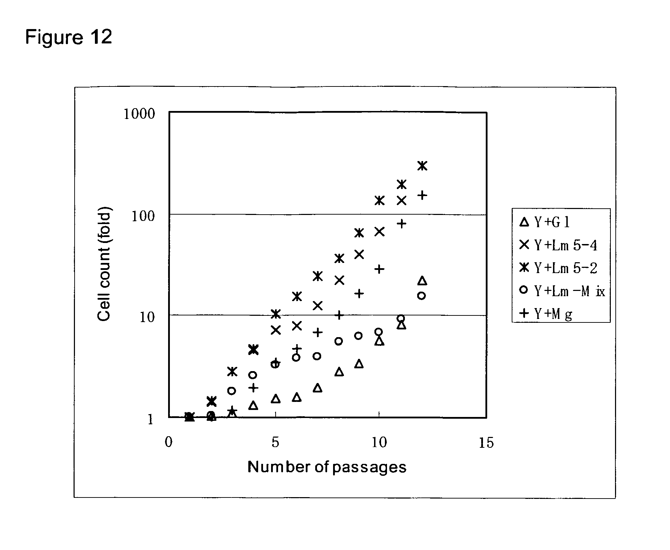

Figure 12 shows a comparison of extracellular matrix proteins for their effect on the prolonged subculture of ES cells in the absence of feeder cells and using a medium (medium Y) supplemented with the serum replacement shown in Example 6. Figure 12 shows the results of proliferation assay, where open triangles represent Y+G1, crosses represent Y+Lm5-4, asterisks represent Y+Lm5-2, open circles represent K+Lm-Mix, and plus signs (+) represent Y+Mg.

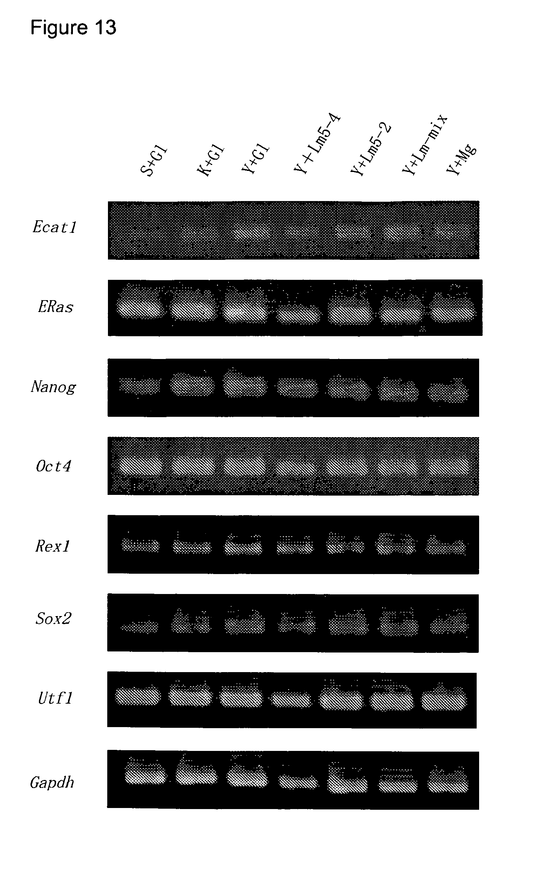

Figure 13 shows the results of RT-PCR compared for the expression of various undifferentiation markers under each culture conditions in ES cells when cultured in medium Y.

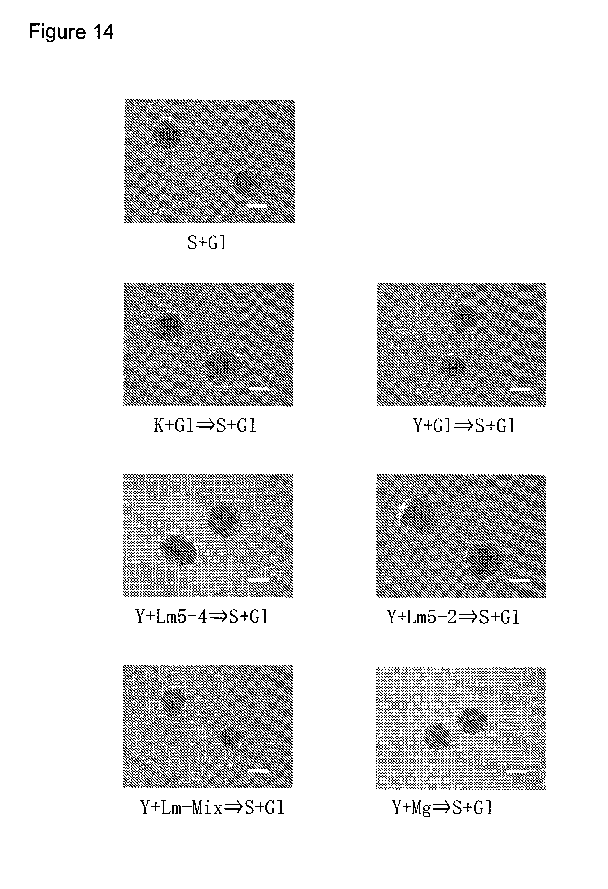

Figure 14 shows embryoid bodies observed for ES cells when cultured in medium Y and then cultured in a LIF-free maintenance medium. In Figure 14, the scale bar represents 250 µm.

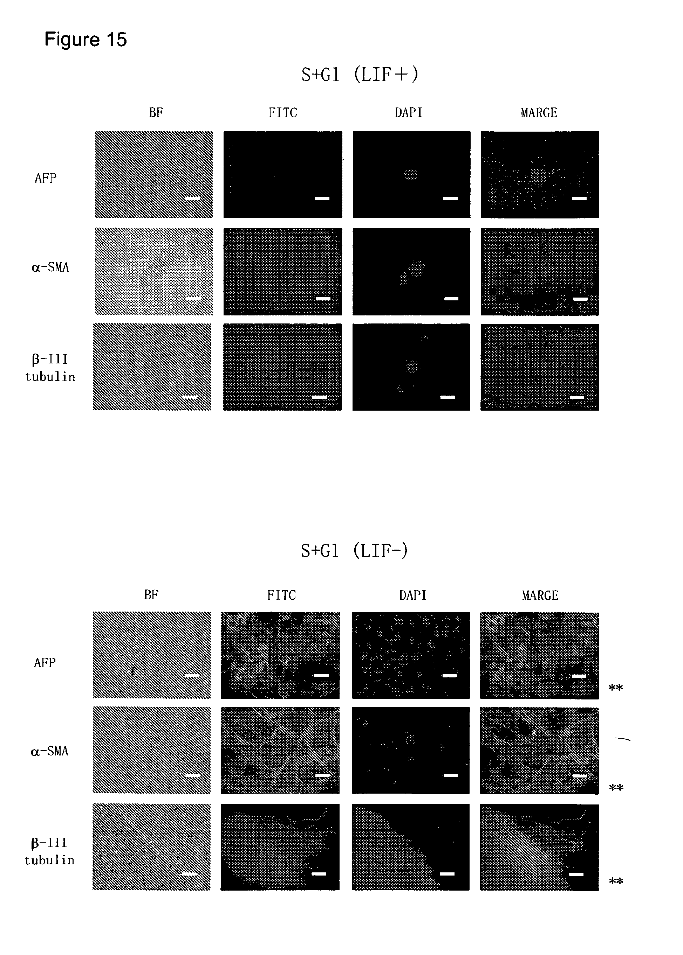

Figure 15 shows the results studied for the differentiation potency of ES cells in a culture system of serum medium on gelatin-coated plates (S+Gl) (Example 7). In Figure 15, the upper panel shows the results of S+Gl (LIF+), while the lower panel shows the results of S+Gl (LIF-). In Figure 15, although the scale bar represents 100 µm, a double asterisk (**) indicates that the scale bar represents 25 µm.

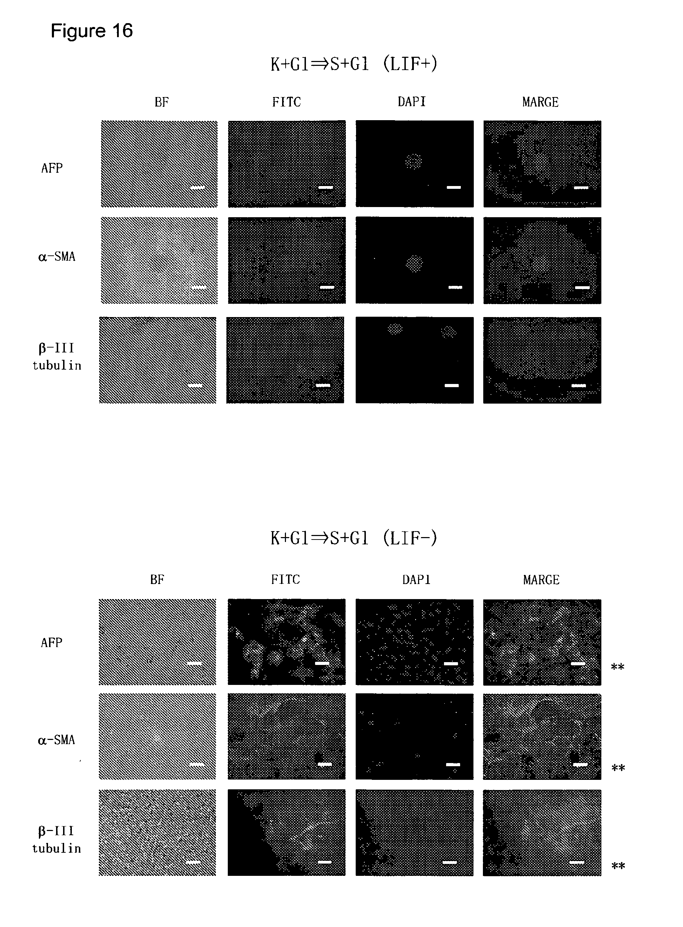

Figure 16 shows the results studied for the differentiation potency of ES cells when cultured in a culture system of KSR-supplemented serum-free medium on gelatin-coated plates (K+Gl) and then returned to a culture system of serum medium (S+Gl) (Example 7). In Figure 16, the upper panel shows the results of K+Gl ⇒ S+Gl (LIF+), while the lower panel shows the results of K+Gl ⇒ S+Gl (LIF-). In Figure 16, although the scale bar represents 100 µm, a double asterisk (**) indicates that the scale bar represents 25 µm.

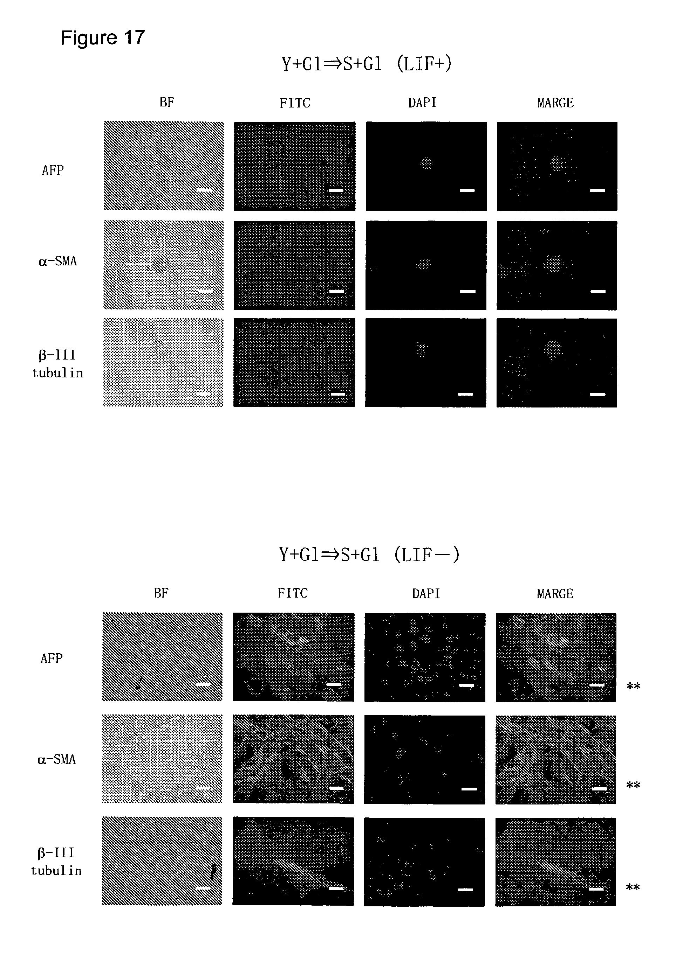

Figure 17 shows the results studied for the differentiation potency of ES cells when cultured in a culture system of medium Y on gelatin-coated plates (Y+Gl) and then returned to a culture system of serum medium (S+Gl). In Figure 17, the upper panel shows the results of Y+Gl ⇒ S+Gl (LIF+), while the lower panel shows the results of Y+Gl ⇒ S+Gl (LIF-). In Figure 17, although the scale bar represents 100 µm, a double asterisk (**) indicates that the scale bar represents 25 µm.

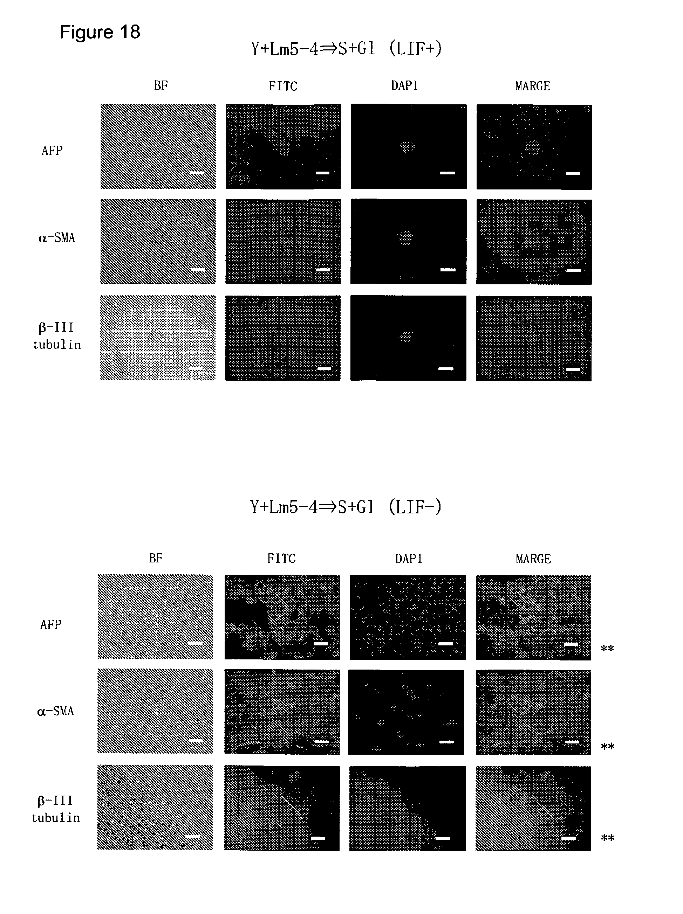

Figure 18 shows the results studied for the differentiation potency of ES cells when cultured in a culture system of medium Y on 4 µg/ml recombinant human laminin-5-coated plates (Y+Lm5-4) and then returned to a culture system of serum medium (S+Gl). In Figure 18, the upper panel shows the results of Y+Lm5-4 ⇒ S+Gl (LIF+), while the lower panel shows the results of Y+Lm5-4 ⇒ S+Gl (LIF-). In Figure 18, although the scale bar represents 100 µm, a double asterisk (**) indicates that the scale bar represents 25 µm.

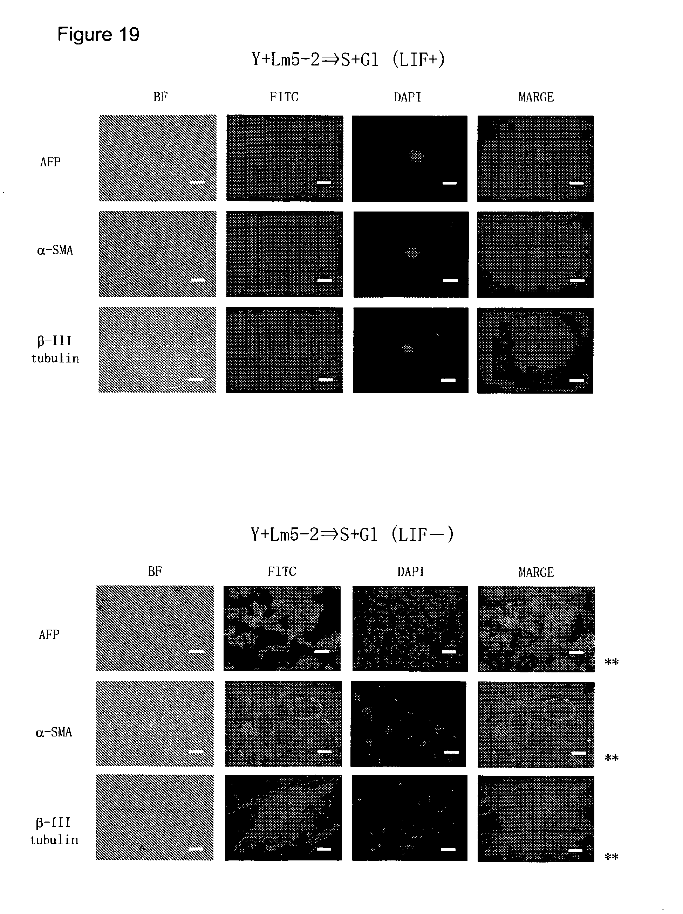

Figure 19 shows the results studied for the differentiation potency of ES cells when cultured in a culture system of medium Y on 2 µg/ml recombinant human laminin-5-coated plates (Y+Lm5-2) and then returned to a culture system of serum medium (S+Gl). In Figure 19, the upper panel shows the results of Y+Lm5-2 ⇒ S+Gl (LIF+), while the lower panel shows the results of Y+Lm5-2 ⇒ S+Gl (LIF-). In Figure 19, although the scale bar represents 100 µm, a double asterisk (**) indicates that the scale bar represents 25 µm.

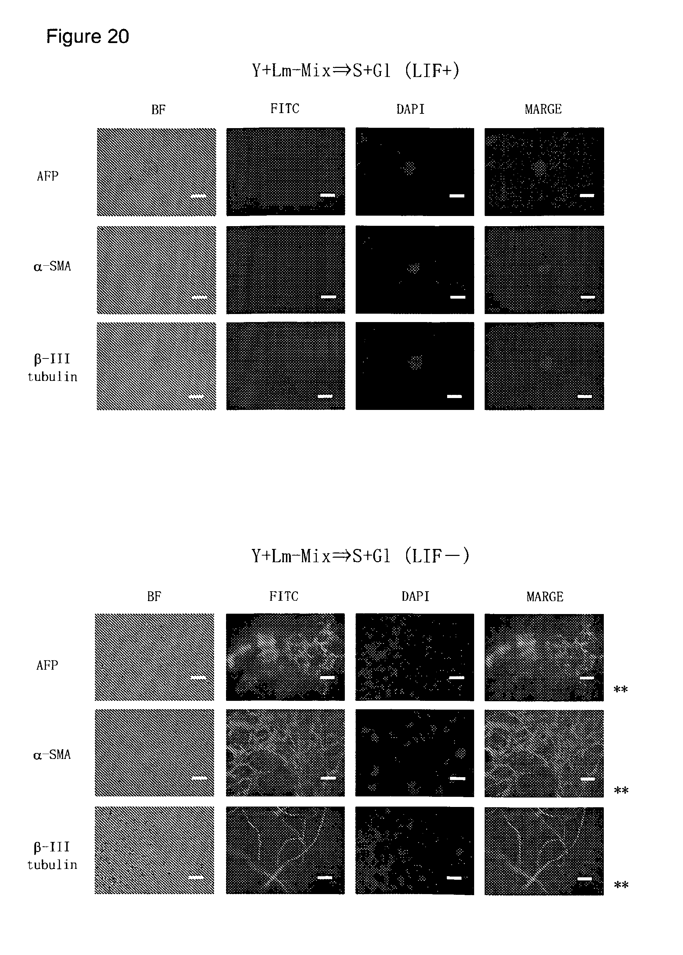

Figure 20 shows the results studied for the differentiation potency of ES cells when cultured in a culture system of medium Y in the presence of Lm-Mix (Y+Lm-Mix) and then returned to a culture system of serum medium (S+Gl). In Figure 20, the upper panel shows the results of Y+Lm-Mix ⇒ S+Gl (LIF+), while the lower panel shows the results of Y+Lm-Mix ⇒ S+Gl (LIF-). In Figure 20, although the scale bar represents 100 µm, a double asterisk (**) indicates that the scale bar represents 25 µm.

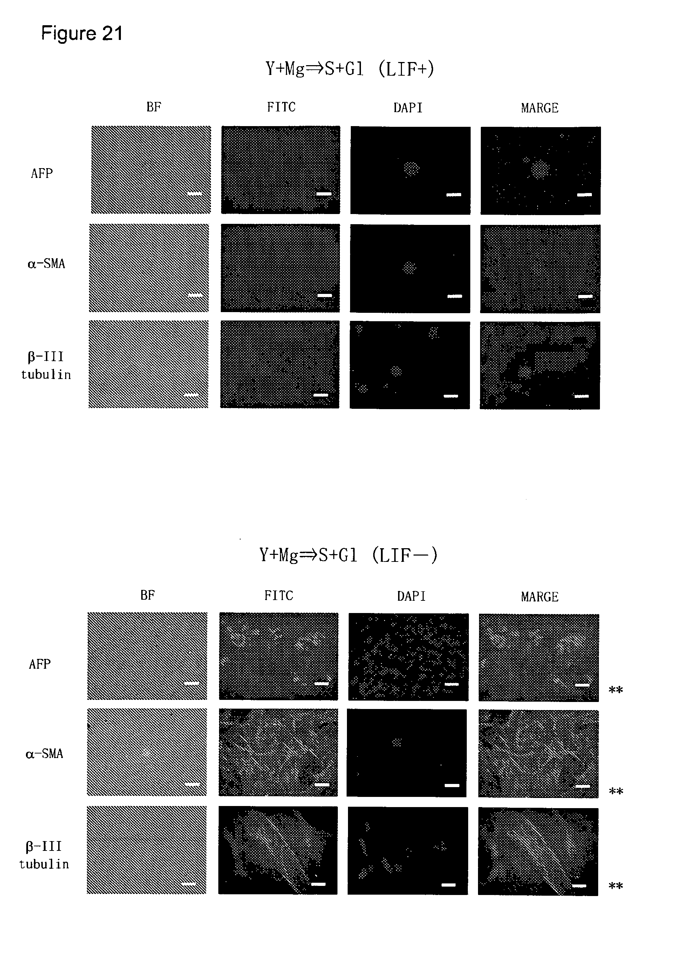

Figure 21 shows the results studied for the differentiation potency of ES cells when cultured in a culture system of medium Y in the presence of Mg (Y+Mg) and then returned to a culture system of serum medium (S+Gl). In Figure 21, the upper panel shows the results of Y+Mg ⇒ S+Gl (LIF+), while the lower panel shows the results of Y+Mg ⇒ S+Gl (LIF-). In Figure 21, although the scale bar represents 100 µm, a double asterisk (**) indicates that the scale bar represents 25 µm.

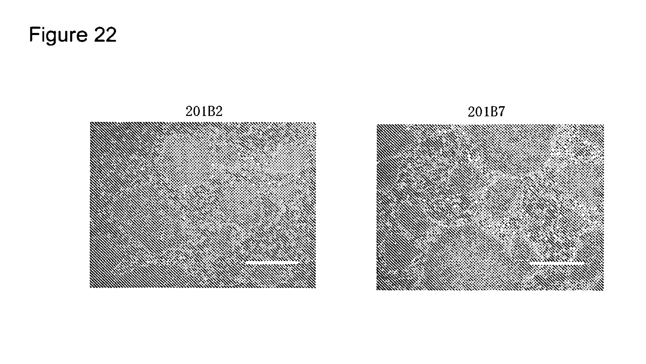

Figure 22 shows the morphology of human iPS cells on feeder cells. In Figure 22, the left panel shows the morphology of 201B2, while the right panel shows the morphology of 201B7. In Figure 22, the scale bar represents 1 mm.

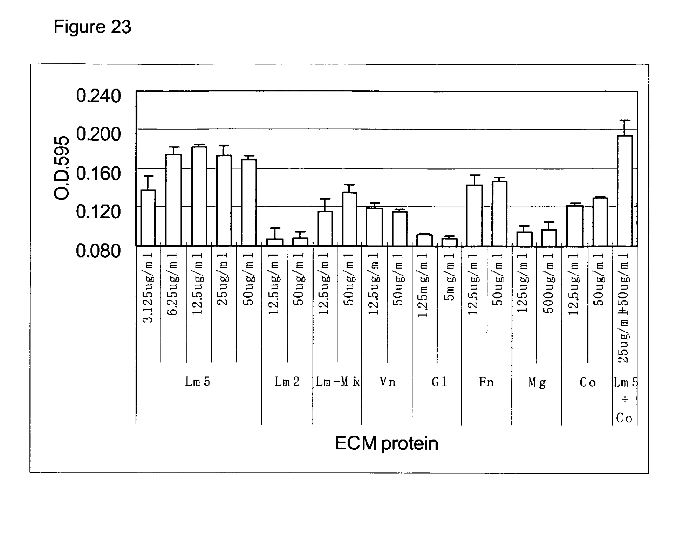

Figure 23 shows a comparison between recombinant human laminin-5 and various extracellular matrix proteins for their effect on the adhesion effect on human iPS cells, as analyzed by adhesion assay.

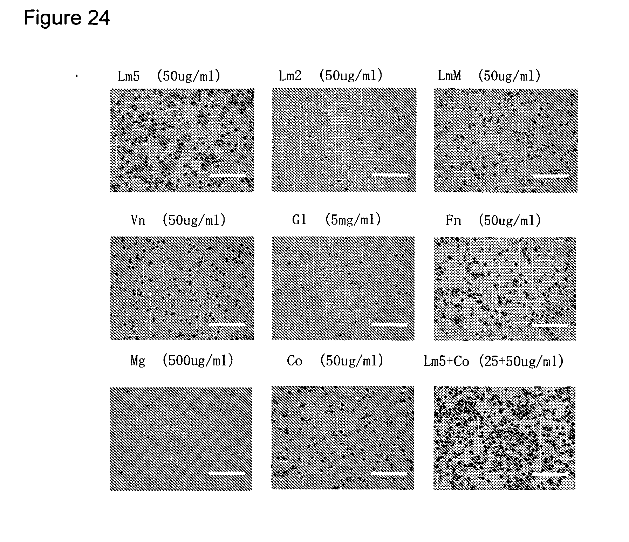

Figure 24 shows the morphology of adhered cells observed in a comparison between recombinant human laminin-5 and various extracellular matrix proteins for their effect on the adhesion effect on human iPS cells, as analyzed by adhesion assay. In Figure 24, the scale bar represents 250 µm.

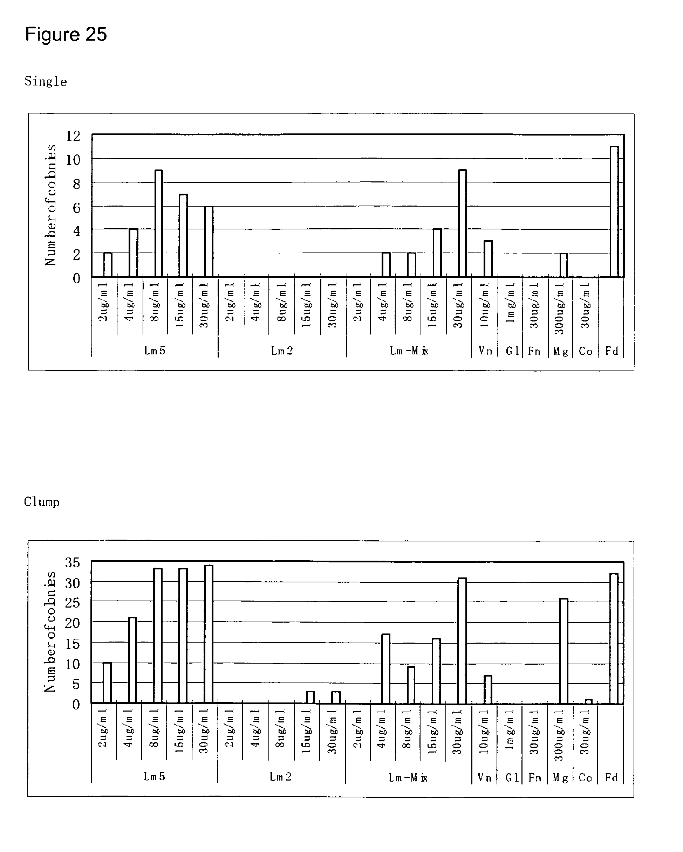

Figure 25 shows a comparison between recombinant human laminin-5 and various extracellular matrix proteins for their effect on the colony formation of human iPS cells, as analyzed by colony assay. In Figure 25, the upper panel shows the results of Single, while the lower panel shows the results of Clump.

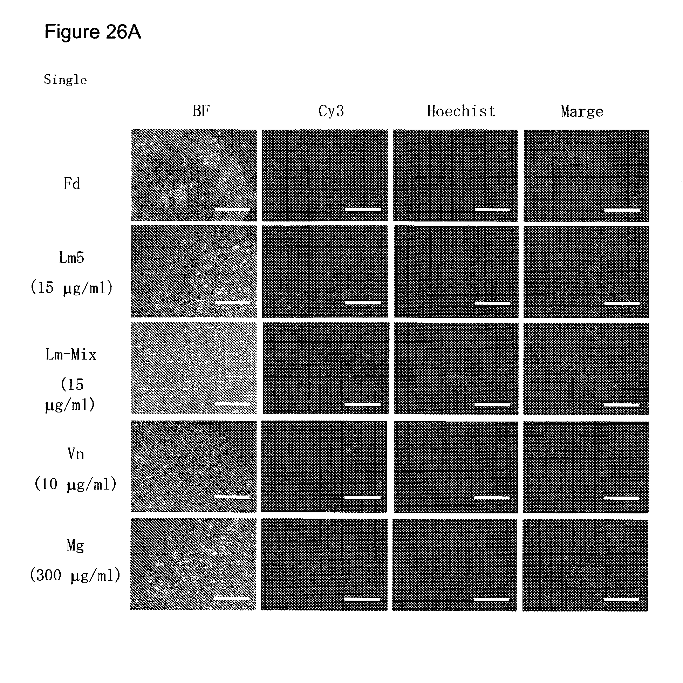

Figure 26A shows the results studied for maintenance of an undifferentiated state in human iPS cell colonies formed from single cells on recombinant human laminin-5 and various extracellular matrix proteins. Figure 26A shows the results of immunostaining obtained after colony assay in Single state, and the scale bar represents 250 µm.

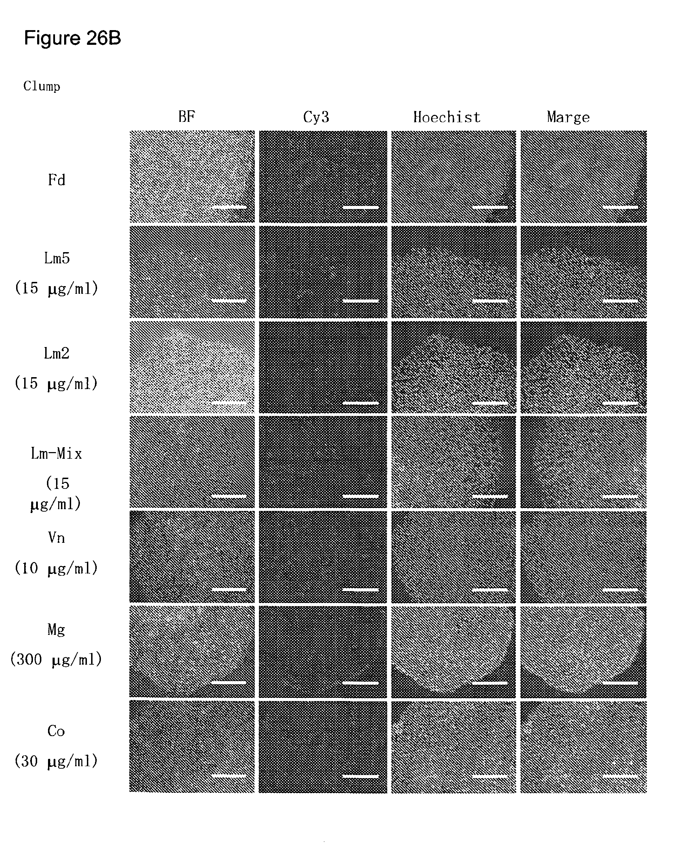

Figure 26B shows the results studied for maintenance of an undifferentiated state in human iPS cell colonies formed from cell clumps on recombinant human laminin-5 and various extracellular matrix proteins. Figure 26B shows the results of immunostaining obtained after colony assay in Clump state, and the scale bar represents 250 µm.

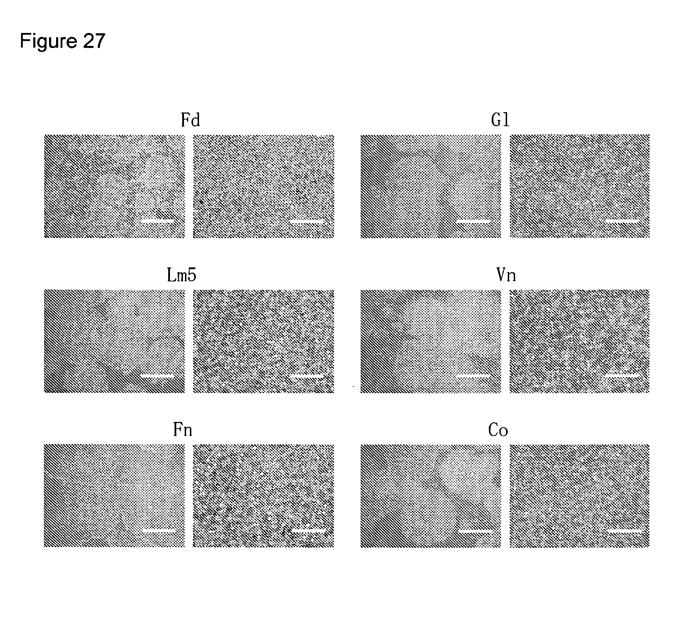

Figure 27 shows the morphology of human iPS cells at 5 weeks of culture during prolonged subculture of human iPS cells formed on recombinant human laminin-5 and various extracellular matrix proteins. The scale bar represents 1 mm (left panel) and 100 µm (right panel) for each extracellular matrix.

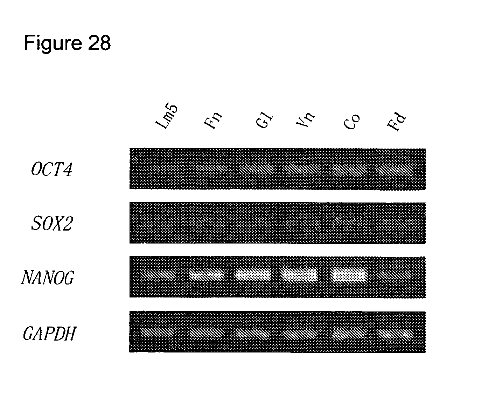

Figure 28 shows the results studied for the expression of various undifferentiation markers in human iPS cells when cultured in the presence of recombinant human laminin-5 and various extracellular matrix proteins.

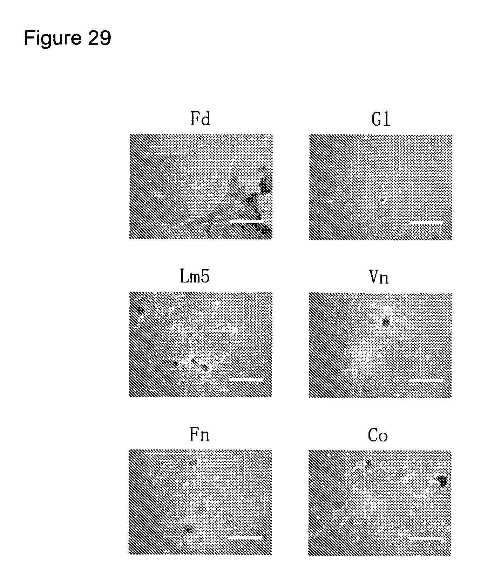

Figure 29 shows the morphology of human iPS cells when induced to differentiate after culture in the presence of recombinant human laminin-5 and various extracellular matrix proteins. In Figure 29, the scale bar represents 1 mm.

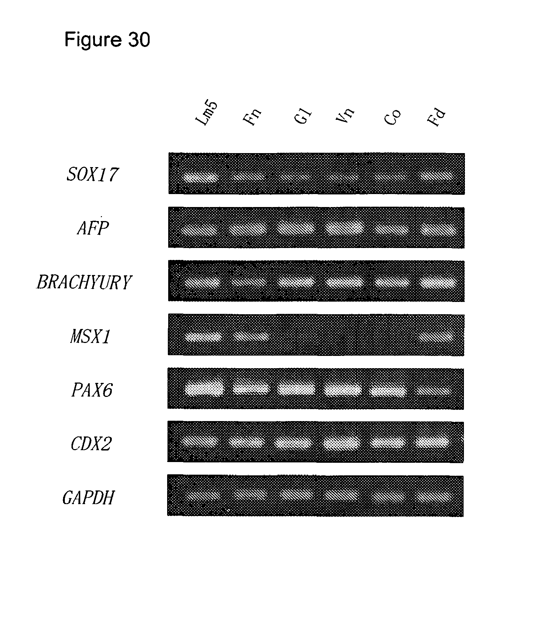

Figure 30 shows the results studied for the expression of various differentiation markers in human iPS cells when induced to differentiate after culture in the presence of recombinant human laminin-5 and various extracellular matrix proteins.

### DESCRIPTION OF EMBODIMENTS

Details and additional features and advantages of the present invention will be described in more detail below on the basis of embodiments.

### 1. Method for proliferation of pluripotent stem cells

The present invention provides a method for proliferation of induced pluripotent stem cells or embryonic stem cells. The method of the present invention comprises culturing those stem cells in a medium free from both feeder cells and serum in a system containing laminin-5.

### Pluripotent stem cells

As used herein, the term "pluripotent stem cells" is intended to collectively refer to stem cells having the ability to differentiate into cells of any tissue type (differentiation pluripotency). Although ES cells are used for study in the Example section described later, pluripotent stem cells that can be proliferated by the method of the present invention include not only embryonic stem cells, but also all induced pluripotent stem cells derived from, e.g., cells of adult mammalian organs or tissues, bone marrow cells, blood cells, and embryonic or fetal cells, as long as their characters are similar to those of embryonic stem cells. In this case, characters similar to those of embryonic stem cells can be defined by cell biological properties specific to embryonic stem cells, including the presence of surface (antigen) markers specific to embryonic stem cells, the expression of genes specific to embryonic stem cells, or the ability to form teratomas.

Specific examples of cells that can be proliferated by the method of the present invention include embryonic stem cells (ES cells), induced pluripotent stem cells (iPS cells), non-human embryonic germ cells (EG cells), non-human germline stem cells (GS cells) and so on. It should be noted that pluripotent stem cells preferred in the present invention are ES cells and iPS cells. iPS cells are particularly preferred because they pose no ethical problem.

As used herein, the term "ES cells" is intended to mean cell lines designed to allow *in vitro* culture, which are prepared from pluripotent stem cells at the early developmental stage having the ability to differentiate into all tissue cells constituting the whole body. ES cells can be expanded to virtually unlimited numbers while retaining their ability to differentiate into all cells constituting the whole body, as in the case of pluripotent stem cells in early embryos.

More specifically, mouse ES cells are the first ES cells reported in 1981 (Proc. Natl. Acad. Sci. USA 78, 7634-7638, 1981, Nature 292, 154-156, 1981). ES cells have pluripotency and can generate all tissue and cell types constituting the whole body.

Pluripotent embryonic stem cells have been isolated from a wide variety of species, including rats (Iannaconns et al., Dev. Biol. 163, 288-292, 1994), hamsters (Dev. Biol. 127, 224-227, 1988), rabbits (Mol. Reprod. Dev. 36, 424-433, 1993), birds, fish, pigs (Reprod. Fertil. Dev. 6, 563-568, 1994), cattle (Reprod. Fertil. Dev. 6, 553-562, 1994), as well as primates (Proc. Natl. Acad. Sci. USA 92, 7844-7848, 1995).

The following should be regarded for information only. In addition, some research teams have succeeded in isolating ES cells and ES cell-like stem cells from embryonic human tissue. Their early successes are as described in Science 282, 1145-1147, 1998, Proc. Natl. Acad. Sci. USA 95, 13726-13731, 1998, Nature Biotech., 18, 399-404, 2000. These ES cell lines have been established from ICM separated from blastocysts by being cultured on feeder cells. Other recent studies have indicated that embryos and embryonic cells can be obtained when nuclei derived from embryos and mature mammalian cells are transplanted into enucleated oocytes.

In 2006, the group of Robert Lanza et al. (Advanced Cell Technology, Inc.) succeeded in establishing mouse and human ES cells by using only single blastomeres from embryos at the cleavage stage before the blastocyst stage in embryonic discs, without impairing the developmental potency of embryos (Nature 439: 216-219, 2006, Cell Stem Cell 2, 113-117, 2008). The development of this technique enabled the establishment of ES cells without destructing fertilized eggs. In the same year, the group of Miodrag Stojkovic et al. (University of Newcastle) succeeded in establishing ES cells from human embryos whose development was arrested (Stem Cells 24, 2669-2676, 2006). This allowed excess eggs to be used, which had been discarded in infertility treatment.

Moreover, in 2004, the group of Yury Verlinsky et al. (Reproductive Genetics Institute of Chicago) succeeded in establishing 20 ES cell lines from human embryos having hereditary diseases. These are the first ES cells that can be used for therapeutic studies on serious hereditary diseases. In addition to them, Yury Verlinsky now possesses 200 or more ES cell lines having different genes, which can be used for screening of pharmaceuticals or other purposes.

In the method of the present invention, established ES cell lines can be used. On the other hand, to avoid immunological rejection which will occur when ES cells prepared by the method of the present invention are applied to an individual, it is effective to use the subject's somatic cells to create clone embryos, from which ES cell lines are then established. This technique allows the establishment of ES cells having the same genetic elements as the individual.

On the other hand, during creation of somatic cell clones, a phenomenon called "reprogramming" would occur, in which somatic cell nuclei introduced into ova would enter the same state as the nuclei of fertilized eggs. ES cells are reported to also have activity similar to such activity as observed in ova (Curr. Biol., 11, 1553-1558, 2001). Namely, it is expected that fusion between individual's somatic cells and ES cells allows the somatic cells to be converted into ES cell-like cells. Since ES cells can be genetically manipulated *in vitro*, it is expected that when ES cells pre-treated to modify factors responsible for immunological rejection (e.g., groups of MHC genes) are used for this purpose, rejection reaction can be avoided without using techniques such as creation of somatic cell clone embryos with the proviso that cloning of human beings is excluded.

In the present invention, "ES cells" are preferably human ES cells. Established human ES cell lines are currently available, for example, from the Institute for Frontier Medical Sciences, Kyoto University.

Alternatively, ES cells can also be prepared as described in the various documents cited herein above.

As used herein, the term "iPS cells" is intended to mean cells having differentiation pluripotency similar to that of ES cells, which are obtained from somatic cells by introducing genes for transcription factors (e.g., Oct3/4, Sox2, Klf4, c-Myc). Thus, as in the case of ES cells, iPS cells can also be expanded to unlimited numbers while retaining their differentiation pluripotency.

To isolate only somatic cells which have been converted into ES-like cells, the group of Shinya Yamanaka et al. (Kyoto University) focused on a gene called Fbx15, which is expressed only in ES cells but is not required for maintenance of their differentiation pluripotency. At this gene locus, they introduced the neomycin resistance gene by homologous recombination techniques and supplemented the medium with G418, which is detoxicated by the action of this resistance gene, to construct an experimental system by which only ES-like cells expressing Fbx15 would acquire G418 resistance and hence survive, whereas normal somatic cells not expressing Fbx15 would be killed. Using this experimental system, they found that 4 genes, Oct3/4, Sox2, Klf4 and c-Myc, were sufficient to establish iPS cells (Cell. 126, 663-672, 2006).

Further, the group of Shinya Yamanaka et al. also succeeded in establishing human iPS cells from fibroblasts by using OCT3/4, SOX2, KLF4 and C-MYC, which are human homologs of the mouse genes used for establishment of mouse iPS cells (Cell. 131, 861-872, 2007).

Concurrently, the group of James Thomson et al., who were the first researchers in the world to establish human ES cells, succeeded in establishing human iPS cells when 4 genes, OCT3/4, SOX2, NANOG and LIN28, among genes expressed specifically in human ES cells were introduced into fetal lung-derived fibroblasts or neonatal foreskin-derived fibroblasts by using the same strategy as that of Shinya Yamanaka et al. (Kyoto University) used for successful establishment of mouse iPS cells (Science 318:1917-1920, 2007).

Moreover, in December 2007, the group of Shinya Yamanaka et al. (Kyoto University) demonstrated that only three factors, Oct-4, Sox2 and Klf4, were sufficient to establish iPS cells in mice and humans without introducing the c-Myc gene, and thus succeeded in suppressing the conversion of iPS cells into cancer cells (Nat. Biotechnol. 26:101-106, 2008). Almost at the same time, the group of Rudolf Jaenisch et al. (Massachusetts Institute of Technology) also succeeded in similar experiments in mice (Cell Stem Cell 2:10-12, 2008).

Moreover, to avoid risks such as carcinogenesis, attempts have been made to further reduce the number of factors to be introduced. As a result, Hans R Scholer et al. (Max Planck Institute for Biochemistry) have succeeded in establishing iPS cells by introducing two genes, i.e., either Oct4 and Klf4 or Oct4 and c-Myc into adult mouse neural stem cells (Nature 454:646-650, 2008). Recently, Hans R Scholer et al. have further reported that the introduction of Oct4 alone is sufficient to prepare iPS cells from neural stem cells (Cell 136:411-419, 2009).

Further, Sheng Ding et al. have reported that some genes can be compensated by small-molecule compounds during induction of iPS cells (Cell Stem Cell 3, 568-574, 2008). They have demonstrated that the use of small-molecule compounds such as BIX and BayK enables the induction of iPS cells by introducing only two genes, Oct4 and Klf4, into mouse embryonic fibroblasts.

Furthermore, attempts have also been made to improve gene transfer techniques for induction of iPS cells. Although viruses (e.g., retrovirus) with a high potential to integrate a transgene into the chromosome are used widely at present, there are reports of techniques using adenovirus (Science 322, 945-949, 2008) or plasmid vectors (Science 322, 949-953, 2008), which appear to be less integrated.

In the present invention, "iPS cells" are preferably human iPS cells. Established human iPS cell lines are currently available, for example, from Kyoto University or RIKEN BioResource Center.

Alternatively, iPS cells may also be prepared by reference to the documents shown below. For example, induced pluripotent stem cells can be prepared according to the procedures described in the documents by the group of Professor Shinya Yamanaka (Kyoto University) (Cell 131, 861-872, 2007, Nat. Biotechnol. 26, 101-106, 2008) or in the document by the group of Thomson (University of Wisconsin) (Science 318, 1917-1920, 2007).

More specifically, any type of somatic cells may be introduced with at least one or more genes selected from Oct3/4, Sox2, c-Myc, Klf4, Nanog and LIN28, and then screened by detecting the expression of genes or proteins specific to pluripotent stem cells to prepare iPS cells.

As in the case of ES cells, the iPS cells thus prepared can be cultured together with basic fibroblast growth factor in the presence of mouse fibroblasts whose proliferation has been inactivated or alternatives thereof, and can also be used as pluripotent stem cells.

These iPS cells have been found to have the same properties as ES cells in relation to features of differentiation into various tissues and features of gene expression in the cells (Cell. 126, 663-672, 2006, Cell 131:861-872, 2007, Science 318, 1917-1920, 2007), and conditions for culturing ES cells and conditions for inducing differentiation from ES cells into various tissues can be applied directly to iPS cells (Takahashi and Yamanaka, Saibo Kogaku (Cell Technology), Vol. 27, No. 3, 252-253, 2008).

As used herein, the term "EG cells" is intended to mean any type of non-human embryonic germ cells prepared from primordial germ cells, and their origin is not limited in any way.

As used herein, the term "GS cells" refers to non-human germline stem cells prepared from germ cells in the testis, i.e., cell lines of spermatogonial stem cells (sperm stem cells) designed to allow *in vitro* culture (Cell. 119, 1001-1012, 2004). Among non-human GS cells, mGS cells (multipotent germline stem cells) are particularly preferred because they have the same properties as ES cells and also have differentiation pluripotency. When used herein, the term "GS cells" means mGS cells, depending on the context.

### Laminin-5

The method of the present invention is directed to the culture of pluripotent stem cells and its most remarkable feature lies in culturing the pluripotent stem cells in a system containing laminin-5.

Laminin-5 is reported to have stronger adhesion activity on many cell types than various extracellular matrix proteins including other laminin isoforms (J. Biochem. 116, 862-869, 1994, J. Cell Biol. 125, 205-214, 1994, Mol Biol Cell. 16, 881-890, 2005).

As shown in Table 1, laminin-5 is a laminin molecule composed of α3, β3 and γ2 chains, which plays a dominant role in binding between epidermis and corium, and binds preferentially to integrin α3β1 in most cells and also binds to integrin α6β1 or α6β4 in some cells. In laminin-5, it has been elucidated that the α3G2A sequence (RERFNISTPAFRGCMKNLKKTS) in the α3 chain G2 domain and the KRD sequence in the G3 domain are major binding sites for integrin.

It is also known that laminin-5, after being secreted as a trimer, receives limited hydrolysis by protease to remove G4 and G5 domains located at the C-terminal of the α3 chain, and is thereby converted from 190 kDa (non-truncated) into 160 kDa (truncated). Laminin-5 isolated in a standard manner does not have G4 and G5 domains. Such α3 chain-truncated laminin-5 is known to have higher stimulatory activities on cell adhesion, motility and neuranagenesis, when compared to non-truncated laminin-5 (J. Biol. Chem., 280 (2005), 14370-14377).

Laminin-5 in the present invention is not limited in any way, and may be either in a non-truncated form containing G4 and G5 domains or in a truncated form free from all or part of G4 and G5 domains.

Moreover, the laminin-5 protein may be either naturally occurring or modified to have one or more modified amino acid residues while retaining its biological activities, particularly stimulatory activity on cell adhesion. Moreover, the laminin-5 protein in the present invention may be of any origin and may be prepared in any manner, as long as it has the features described herein. Namely, the laminin-5 protein of the present invention may be naturally occurring, expressed from recombinant DNA by genetic engineering procedures, or chemically synthesized.

The laminin-5 protein may be of any origin, preferably of human origin. In a case where human pluripotent stem cells are cultured in order to obtain materials for regenerative medicine, etc., it is preferred to use laminin-5 of human origin in the sense of avoiding the use of materials derived from other animals.

SEQ ID NOs: 1 to 6 in the Sequence Listing herein show the nucleotide and amino acid sequences of human laminin-5 α3, β3 and γ2 chains, respectively. The laminin-5 protein to be used in the present invention is preferably a protein composed of the following subunits: an α3 chain having the amino acid sequence of SEQ ID NO: 2 or an amino acid sequence comprising deletion, addition or substitution of one or more amino acids in the sequence of SEQ ID NO: 2 (amino acid residues 1-1713) (J. Biol. Chem. 269, 22779-22787, 1994), a β3 chain having the amino acid sequence of SEQ ID NO: 4 or an amino acid sequence comprising deletion, addition or substitution of one or more amino acids in the sequence of SEQ ID NO: 4 (amino acid residues 1-1170) (J. Biol. Chem. 269, 11073-11080, 1994), and a γ2 chain having the amino acid sequence of SEQ ID NO: 6 or an amino acid sequence comprising deletion, addition or substitution of one or more amino acids in the sequence of SEQ ID NO: 6 (amino acid residues 1-1193) (J. Cell. Biol. 119, 679-693, 1992).

Globular domains (G1 to G5 domains) in the α3 chain correspond to amino acid residues 794-970, 971-1139, 1140-1353, 1354-1529 and 1530-1713, respectively, in SEQ ID NO: 1.

Each chain of laminin-5 may have an amino acid sequence comprising deletion, addition or substitution of one or more amino acid residues in the amino acid sequence shown in the corresponding SEQ ID NO. Such proteins having amino acid sequences homologous to naturally occurring proteins can also be used in the present invention. The number of amino acids which may be modified is not limited in any way in the respective amino acid sequences of α3, β3 and γ2 chains, but it is preferably 1 to 300 amino acid residues, 1 to 200 amino acid residues, 1 to 150 amino acid residues, 1 to 120 amino acid residues, 1 to 100 amino acid residues, 1 to 80 amino acid residues, 1 to 50 amino acid residues, 1 to 30 amino acid residues, 1 to 20 amino acid residues, 1 to 15 amino acid residues, 1 to 10 amino acid residues, or 1 to 5 amino acid residues. More preferred is a possible number of amino acid residues which may be modified by known site-directed mutagenesis, for example, 1 to 10 amino acid residues, or 1 to 5 amino acid residues.

It is well known in the art that conservative substitution of amino acids can be used to obtain proteins or polypeptides retaining their original functions. Such substitution includes replacement of an amino acid with another residue having similar physical and chemical properties, as exemplified by replacement of one fatty acid residue (Ile, Val, Leu or Ala) with another, or replacement between basic residues Lys and Arg, between acidic residues Glu and Asp, between amide residues Gln and Asn, between hydroxyl residues Ser and Tyr, or between aromatic residues Phe and Tyr.

Laminin-5 to be used in the present invention may also be a protein sharing at least 80%, 85%, 90%, 95%, 98% or 99% identity with the amino acid sequences shown in SEQ ID NOs: 2, 4 and 6 and having the ability to stimulate cell adhesion activity. When the structure of constituent subunits is compared between laminin-5 and laminin-1, the amino acid sequence homology in each subunit is 50% or less. In particular, the homology in the above α chain G domains is as low as about 25%.

Identity is calculated as follows: the number of identical residues is divided by the total number of residues in a corresponding known sequence or a domain therein, and then multipled by 100. Computer programs available for use in the determination of sequence identity using standard parameters include, for example, Gapped BLAST PSI-BLAST (Nucleic Acids Res. 25, 3389-340, 1997), BLAST (J. Mol. Biol. 215:403-410, 1990), and Smith-Waterman (J. Mol. Biol. 147:195-197, 1981). In these programs, default settings are preferably used, but these settings may be modified, if desired.

The laminin-5 protein in the present invention may be of any origin and may be prepared in any manner, as long as it has the features described herein. Namely, the laminin-5 protein of the present invention may be a naturally occurring laminin-5 protein as found in or purified from the supernatant of human or animal cells secreting laminin-5. However, laminin-5 can be effectively produced as a gene recombinant protein by expressing each subunit using recombinant DNA technology known in the art. It is particularly preferred to obtain laminin-5 as a human recombinant protein, in the sense of avoiding unwanted factors derived from other animals.

For this purpose, primers may be designed based on a DNA sequence comprising nucleic acid residues 1-5139 in SEQ ID NO: 1 (encoding the laminin-5 α3 chain) and nucleotide sequences of nucleic acid residues 121-3630 in SEQ ID NO: 3 (encoding the β3 chain) and nucleic acid residues 118-3696 in SEQ ID NO: 5 (encoding the γ2 chain), and an appropriate cDNA library may be used as a template in polymerase chain reaction (PCR) to amplify desired sequences. Such PCR procedures are well known in the art and can be found, e.g., in "PCR Protocols, A Guide to Methods and Applications," Academic Press, Michael, et al., 1990.

DNA encoding a gene for each chain of laminin-5 may be integrated into an appropriate vector and then introduced into either eukaryotic or prokaryotic cells by using an expression vector that allows expression in each host, whereby the respective chains are expressed to obtain a desired protein. Host cells which can be used to express laminin-5 are not limited in any way and include prokaryotic host cells such as *E. coli* and *Bacillus subtilis*, as well as eukaryotic hosts such as yeast, fungi, insect cells and mammalian cells. It should be noted that human fetal kidney cell line HEK293 used in the Example section described later is particularly preferred as a host cell.

A vector constructed to express laminin-5 can be introduced into the above host cells by transformation, transfection, conjugation, protoplast fusion, electroporation, particle gun technique, calcium phosphate precipitation, direct microinjection or other techniques. The cells containing the vector may be grown in an appropriate medium to produce a laminin-5 protein to be used in the present invention, which may then be purified from the cells or medium to obtain the laminin-5 protein. Purification may be accomplished, for example, by size exclusion chromatography, HPLC, ion exchange chromatography, immunoaffinity chromatography, etc.

Laminin-5 is described in detail in JP 2001-172196 A, which is incorporated herein by reference.

### Method for proliferation of pluripotent stem cells

In the present invention, pluripotent stem cells are cultured in a system containing laminin-5. As used herein, the phrase "system containing laminin-5" is intended to mean that a culture system for pluripotent stem cells contains laminin-5 in some fashion, and embodiments thereof are not limited in any way.

In the present invention, it is a preferred embodiment to use a laminin-5-treated culture vessel for culture of pluripotent stem cells in a system containing laminin-5. However, the "system containing laminin-5" intended in the present invention is not limited to this embodiment, and also includes other embodiments where laminin-5 is added to the medium for culture of pluripotent stem cells.

As used herein, the phrase "laminin-5-treated culture vessel" is intended to mean a culture vessel whose surface is treated with laminin-5, e.g., by coating. The "culture vessel" intended in the present invention is not limited in any way, and a vessel of any material and any shape may be used as long as it is sterilized to avoid bacterial contamination and is suitable for cell culture. Examples of such a culture vessel include, but are not limited to, culture dishes, culture flasks, culture petri dishes, culture plates (e.g., 96-well, 48-well, 12-well, 6-well, 4-well plates), culture bottles and so on, all of which are commonly used in the art. Techniques for coating laminin over the surface of a culture vessel are known in the art, and those skilled in the art would be able to select any type of culture vessel suitable for the object of the present invention, treat the vessel with laminin-5, and use the laminin-5-treated vessel to culture pluripotent stem cells by the method of the present invention.

The amount of laminin-5 used for culture vessel treatment is not limited in any way. When treated with a laminin-5 solution of 0.05 µg/ml or more, preferably 0.5 to 15 [µg/ml, more preferably 3.75 to 15 µg/ml, good results are obtained.

As shown in the Example section described herein later, the inventors of the present invention have found that recombinant human laminin-5 shows stronger adhesion activity on mouse ES cells than Matrigel, a laminin mixture or other extracellular matrix proteins, and thus have arrived at the present invention. Further, in the present invention, it has been found that mouse ES cells can be maintained and cultured on plates coated with recombinant human laminin-5, even in a LIF-free and serum-free medium and in the absence of MEFs. The culture of mouse ES cells in the absence of MEFs has been conventionally accomplished by using gelatin-coated plates and in the presence of bovine fetal serum (FBS). When cultured by the method of the present invention, mouse ES cells were confirmed to proliferate at a level equal to that in conventional methods even under MEF-free and FBS-free conditions and also to retain their undifferentiated state. In addition, when cultured by the method of the present invention, mouse ES cells were found to retain pluripotency.

Studies were also conducted on human iPS cells, indicating that recombinant human laminin-5 also showed strong adhesion activity on human iPS cells, as demonstrated in the Example section described later. In addition, when cultured on plates coated with recombinant human laminin-5, human iPS cells were found to form colonies even under serum-free conditions, thus indicating that they were able to be maintained and cultured under serum-free conditions. Furthermore, human iPS cell cultured by the method of the present invention were found to retain their undifferentiated state.

Thus, the method of the present invention comprises culturing pluripotent cells in a medium free from both feeder cells and serum, more preferably in a medium free from any substances derived from non-human animals.

For human ES cells, various extracellular matrix proteins (e.g., Matrigel, fibronectin, laminin-1, type 1 collagen, type 4 collagen) were tested in the past as supporting materials alternative to MEFs, but the adhesion activity was as low as a few percent or less in each case (Stem Cell. 24, 2649-2660, 2006). In the present invention, by applying the strong adhesion activity of laminin-5 to maintenance culture of pluripotent stem cells (e.g., human ES cells, human iPS cells), which are very poor in cell adhesion efficiency, improvements can be achieved in both adhesion efficiency and proliferation efficiency in the absence of MEFs. Moreover, to ensure the use of human ES cells or human iPS cells for regenerative medicine, recombinant human laminin-5 is also useful as a material constructing a culture system completely free from animal-derived substances.

Thus, in the present invention, treatment with laminin-5 allows an improvement in the adhesion efficiency of pluripotent stem cells onto a culture vessel and also enables efficient proliferation of pluripotent stem cells without the need to use feeder cells generally required for their culture.

As used herein, the term "feeder cells" is intended to mean additional cells playing a role as an aid, which are used to adjust culture conditions for target pluripotent stem cells to be proliferated or differentiated. In the case of pluripotent cells such as ES cells or iPS cells, in commonly used conventional methods, mouse-derived primary cultured fibroblasts are used as feeder cells and nutrients such as growth factors are supplied from the feeder cells to the pluripotent cells, whereby the pluripotent cells can be cultured. According to the present invention, the strong adhesion efficiency of laminin-5 allowed the culture of pluripotent stem cells such as ES cells or iPS cells without the need to use these feeder cells.

As used herein, the term "supporting material" is intended to mean a proteinous factor used to aid cell proliferation, and laminin-5 is used as a supporting material in the present invention. As shown in the Example section described later, laminin-5 has higher adhesion ability than various extracellular matrixes and is excellent as a supporting material for pluripotent stem cells.

When used herein to describe pluripotent stem cells, the term "differentiation" is intended to mean a change that causes the pluripotent stem cells to lose their differentiation pluripotency (i.e., potential ability to differentiate into all tissues) and to have characters as cells constituting a specific tissue. For example, undifferentiation markers of pluripotent stem cells, such as Ecat1, ERas, Nanog, Oct4, Rex1, Sox2 and Utf1, may be measured to thereby evaluate whether pluripotent stem cells do not differentiate during culture. As shown in Examples 3 and 4 described later, ES cells cultured by the method of the present invention were evaluated for passage-induced differentiation, indicating that they did not differentiate and remained in an undifferentiated state even after 10 or more passages of subculture. Further, as shown in Example 5 described later, ES cells cultured by the method of the present invention were evaluated for maintenance of pluripotency during subculture, indicating that they also retained pluripotency even after passages.

Furthermore, as shown in Example 11 described later, human iPS cells cultured by the method of the present invention were also found not to differentiate and to remain in an undifferentiated state even after subculture for 5 weeks. Moreover, as shown in Example 12 described later, human iPS cells cultured by the present invention were also able to be induced to differentiate.

In one embodiment of the present invention, a culture vessel may be treated with laminin-5, for example, by applying laminin-5 onto the inner surface of the culture vessel and then drying. Such a laminin-5-treated culture vessel is charged with medium (e.g., GMEM or DMEM) commonly used for culture of pluripotent stem cells, and pluripotent stem cells are added to the medium. Then, the pluripotent stem cells are cultured under known appropriate culture conditions, for example but not limited to, under gas phase conditions of 37°C and 5% CO₂.

In a preferred embodiment of the present invention, it is more preferable to add appropriate additives to the culture medium, in addition to the use of a system containing laminin-5. Such additives are preferably those other than serum, and more preferably exclude substances derived from non-human animals.

A non-limiting example of such additives is a serum replacement. A serum replacement is an artificial liquid composition designed to have ingredients similar to those of serum, and it allows cells to proliferate even in the absence of serum. As an example, it is possible to use a serum replacement comprising various amino acids, inorganic salts, vitamins, albumin, insulin, transferrin, and antioxidative ingredients. Various amino acids include, for example, glycine, L-alanine, L-asparagine, L-cysteine, L-aspartic acid, L-glutamic acid, L-phenylalanine, L-histidine, L-isoleucine, L-lysine, L-leucine, L-glutamine, L-arginine, L-methionine, L-proline, L-hydroxyproline, L-serine, L-threonine, L-tryptophan, L-tyrosine, and L-valine. Inorganic salts include, for example, AgNO₃, AlCl₃·6H₂O, Ba(C₂H₃O₂)₂, CdSO₄·8H₂O, CoCl₂·6H₂O, Cr₂(SO₄)₃·1H₂O, GeO₂, Na₂SeO₃, H₂SeO₃, KBr, Kl, MnCl₂·4H₂O, NaF, Na₂SiO₃·9H₂O, NaVO₃, (NH₄)₆Mo₇O₁₄·4H₂O, NiSO₄·6H₂O, RbCl, SnCl₂, ZrOCl₂·8H₂O, and sodium selenite. Vitamins include, for example, thiamine and ascorbic acid. Antioxidative ingredients include, for example, reduced glutathione.

Likewise, Knockout^{™} serum replacement (KSR) is a serum replacement for ES cells, which is commercially available from Invitrogen, Corp. As shown in Examples 3 and 4 described later, it is a preferred embodiment of the present invention that pluripotent stem cells are cultured in a medium supplemented with about 10% KSR.

Further, it is also a preferred embodiment of the present invention to use a serum replacement of the composition shown in Example 6 described later. In Example 6, the detailed composition of a serum replacement is disclosed, which comprises amino acids (e.g., glycine, histidine, isoleucine, methionine, phenylalanine, proline, hydroxyproline, serine, threonine, tryptophan, tyrosine, valine), vitamins (e.g., thiamine, ascorbic acid), trace metal elements (e.g., silver, aluminum, barium, cadmium, cobalt, chromium, germanium, manganese, silicon, vanadium, molybdenum, nickel, rubidium, tin, zirconium), halogen elements (e.g., bromine, iodine, and fluorine), as well as other ingredients (e.g., albumin, reduced glutathione, transferrin, insulin, sodium selenite). However, ingredients constituting the serum replacement intended in the present invention are not limited to those listed above, and various modifications may be made, for example, by replacement with other similar ingredients. Moreover, the content of each ingredient contained in the serum replacement is not limited to that shown in Example 6, and may be adjusted as appropriate depending on the properties of cells and/or the purpose of experiments.

Any serum replacement may be used as long as it has ingredients and functions similar to those of KSR.

### 2. Use of laminin-5 as a cell-supporting material

The present invention is also directed to the use of laminin-5 as a cell-supporting material for proliferation of pluripotent stem cells. As has been described above, laminin-5 has a strong effect on cell adhesion and is therefore useful in culturing pluripotent stem cells in a medium free from both feeder cells and serum.

A serum replacement is preferably Knockout^{™} serum replacement (KSR). Alternatively, it is also possible to use a serum replacement of the composition shown in Example 6.

Examples of additives include nonessential amino acids, sodium pyruvate, mercaptoethanol, antibiotics and so on.

### EXAMPLES

The present invention will now be described in more detail below on the basis of the following examples, which are not intended to limit the scope of the invention.

### Example 1: Preparation of recombinant human laminin-5 (rLm5)

In this example, a recombinant human laminin-5 protein was prepared in a known manner.

From human fetal kidney cell line HEK293 modified to carry cDNAs for α3 chain (SEQ ID NO: 1), β3 chain (SEQ ID NO: 3) and γ2 chain (SEQ ID NO: 3) (Lm5-HEK293), the serum-free supernatant was collected and centrifuged at 4°C at 3000 rpm for 5 minutes. The human fetal kidney cell line HEK293 was obtained as described in J. Biochem. 132, 607-612 (2002). The supernatant was then applied to Heparin sepharose CL-6B (GE healthcare) and eluted. The rLm5-containing fractions were passed through an antibody column, in which mouse anti-Lm-α3 (anti-laminin α3) monoclonal antibody (BG5) was covalently bonded to ProteinA sepharose CL-6B (GE healthcare), and then eluted. It should be noted that monoclonal antibody BG5 is an antibody prepared by the inventors of the present invention using an N-terminal fragment of the laminin α3B chain as an antigen according to known procedures for monoclonal antibody preparation.

Purified rLm5 (1 µg) was denaturated under reducing conditions and then subjected to SDS polyacrylamide gel electrophoresis on a 5-20% gel to confirm the size and purity of α3, β3 and γ2 chains. As a result, bands of 160 kDa, 135 kDa and 105 kDa were detected, respectively. Figure 1 shows a photograph of SDS polyacrylamide gel electrophoresis obtained for purified rLm5.

When analyzed with a CS-Analyzer, purified rLm5 was found to have a purity of about 98%. rLm5 thus prepared was used in the following examples.

### Example 2: Adhesion assay using mouse ES cell line EB3

This example shows the results of adhesion assay using mouse ES cell line EB3, obtained with the use of various cell-supporting materials.

GMEM (SIGMA) supplemented with 10% fetal bovine serum (FBS), 0.1 mM nonessential amino acids (Gibco), 1 mM sodium pyruvate (Gibco), 1000 U/ml ESGRO (Chemicon) and 10⁻⁴ M 2-mercaptoethanol (WAKO) was used as a maintenance medium for mouse ES cell line, EB3 cells. The EB3 cells were provided by the Division of Stem Cell Regulation Research, Area of Molecular Therapeutics, Course of Advanced Medicine G6, Graduate School of Medicine, Osaka University.

In the adhesion assay, a serum-free medium was used, whose composition was the same as that of the maintenance medium, except for not containing FBS. The cell-supporting materials used were bovine gelatin (SIGMA), rLm5, Matrigel^{®} (BD), human vitronectin (SIGMA), human type IV collagen (BD), human fibronectin (BD), human laminin-2 (Chemicon) and human laminin (SIGMA). Further, plates were prepared by being treated with these respective cell-supporting materials, and were subjected to adhesion assay for each cell-supporting material, as described below.

96-well plates (Coming) were treated with various extracellular matrix proteins prepared at desired concentrations, and then blocked with a 1.2% BSA (SIGMA) solution at 37°C for 1 hour. The various extracellular matrix proteins were prepared at the following concentrations: 1.5 mg/ml for gelatin (Gl), 3.75 µg/ml for laminin-2 (Lm2), 3.75 µg/ml for a laminin mixture (Lm-Mix), 150 µg/ml for Matrigel (Mg), 15 µg/ml for collagen (Co), 15 µg/ml for fibronectin (Fn), and 15 µg/ml for vitronectin (Vn). On the other hand, rLm5 (Lm5) was prepared by two-fold serial dilution at three concentrations: 3.75 µg/ml, 7.5 µg/ml and 15 µg/ml.

After washing with the serum-free medium, EB3 cells were seeded at 30000 cells/well in the plates and cultured at 37°C under gas phase conditions of 5% CO₂ and 95% air for 30 or 60 minutes. After culture, the plates were gently shaken with a vortex mixer to release less adhered cells from the plate surface, followed by treatment with Percoll (GE healthcare) to remove these cells. The adhered cells were fixed with 25% glutaraldehyde (Nacalai) and stained with 2.5% crystal violet (Nacalai) for comparison of relative cell counts.

Figure 2 shows the results evaluated for the effect of the various extracellular matrix proteins on EB3 cell adhesion after culture for 30 or 60 minutes, as analyzed by OD595 measurement. The results shown in Figure 2 indicated that rLm5 showed stronger adhesion activity on EB3 cells than the other various extracellular matrix proteins including Lm-Mix.

It should be noted that human ES cells, which are poor in adhesion efficiency, are known to achieve an adhesion efficiency of less than 1% in the case of using EHS-derived laminin, and their adhesion efficiency is as low as 3% even in the case of Matrigel (50-60% EHS-derived laminin, 30% type IV collagen, 10% entactin) which is most widely used at present. In this example, laminin-5 was found to show strong adhesion activity on mouse ES cells, when compared to Matrigel composed primarily of EHS laminin, laminin-2, and placenta-derived laminin (Lm-Mix) deemed to be rich in laminin-10/11. This is regarded as a remarkable effect of laminin-5 used in the present invention.

### Example 3: Proliferation assay using mouse ES cell line EB3

This example shows the results of proliferation assay using mouse ES cell line EB3, obtained with the use of various cell-supporting materials.

The maintenance medium used for EB3 cells was the same as that of Example 2. In the proliferation assay, a medium supplemented with 10% Knockout^{™} serum replacement (KSR, Invitrogen) in place of 10% FBS was used (KSR-GMEM). In 12-well plates (NUNC) which had been treated with various extracellular matrix proteins prepared at desired concentrations, EB3 cells were seeded at 40000 cells/well. After culture at 37°C under gas phase conditions of 5% CO₂ and 95% air for 2 days, the cells were collected by enzyme treatment and counted with a hemacytometer.

The EB3 cells were seeded again at 40000 cells/well in 12-well plates which had been treated with the same various extracellular matrix proteins prepared at desired concentrations. By repeating this procedure, the various extracellular matrixes were compared for their proliferative effect on EB3 cells. The culture medium used was maintenance medium (S) or KSR-GMEM (K). The cell-supporting materials were prepared at the following concentrations: 1 mg/ml for Gl, 4 µg/ml for rLm5 (Lm5-4), 2 µg/ml for rLm5 (Lm5-2), 4 µg/ml for Lm-Mix, and 150 µg/ml for Mg. In this experiment, the EB3 cells were acclimated before use by having been subcultured for several passages in KSR-GMEM.

Figure 3 shows the results studied for the effect of the various extracellular matrixes on EB3 cell proliferation upon culture in the absence of feeder cells in the maintenance medium (S) or KSR-GMEM (K). The results shown in Figure 3 indicated that during the first 2 or 3 passages, K+Lm-Mix and K+Mg resulted in slower proliferation than the other experimental groups.

For this reason, all the experimental groups except for K+Lm-Mix and K+Mg were further subcultured to study the effect of the extracellular matrixes on EB3 cell proliferation upon prolonged subculture in the absence of feeder cells. The theoretical fold increase in the number of cells finally proliferated was calculated and the results obtained are shown in Figure 4. The results shown in Figure 4 indicated that rLm5 showed a proliferative effect on EB3 cells at the same level as the control group (S+Gl), i.e., a widely used conventional maintenance culture system for ES cells.

Figure 5 shows the morphology of EB3 cells upon prolonged subculture in a system of maintenance culture (S+Gl), a system of rLm5 (K+Lm5) and a system of rLm5 followed by maintenance culture (K+Lm5-4 → S+Gl). As shown in Figure 5, the experimental group of K+Lm5 showed a gradual change into epithelial cell-like morphology, but colony formation was observed again when returned to a system of normal maintenance culture (K+Lm5-4 → S+Gl). In general, undifferentiated ES cells are known to form colonies. Thus, the above results suggested that EB3 cells retained their undifferentiated state even when their morphology was changed to epithelial cell-like morphology upon prolonged subculture under experimental conditions of K+Lm5.

### Example 4: Detection of undifferentiation markers

In this example, Ecat1, ERas, Nanog, Oct4, Rex1, Sox2 and Utf1, which are known as undifferentiation markers of mouse ES cells, were measured by RT-PCR on their genes to study whether rLm5 had the effect of allowing EB3 cells to remain in an undifferentiated state.

From the EB3 cells subcultured for 10 or more passages in Example 3, total RNA was extracted using TRIZOL (Invitrogen). After extraction, a ThermoScript RT-PCR System (Invitrogen) was used to synthesize cDNA by reverse transcription reaction. The synthesized cDNA was used as a template to perform PCR with the primers shown in Table 2. Each gene was denatured at 94°C for 30 seconds, annealed for 30 seconds, and elongated at 72°C for 20 seconds. Annealing was performed at a temperature of 64°C for Ecat1, 61°C for ERas, Oct4 and Utf1, 59°C for Rex1, and 54°C for Nanog and Sox2.

Figure 6 shows the results studied for the expression of various undifferentiation markers in each culture. As a result, even under experimental conditions where the cells were cultured on rLm5-coated plates in the presence of KSR (K+Lm5-4, K+Lm5-2), EB3 cells were found to express all the undifferentiation markers analyzed, at the same levels as observed under experimental conditions normally maintained (S+Gl). Moreover, when returned to a system of normal maintenance culture (K+Lm5-4 → S+Gl), the expression of these undifferentiation markers was found to return to levels indistinguishable from those observed when the cells were continued to be cultured in the system of normal maintenance culture (S+Gl). The above results suggested that EB3 cells retained their undifferentiated state even after 10 or more passages of subculture in K+Lm5.

**[Table 2]**

| | RT-PCR primers |
|---|---|
| Ecat1 | |
| | 5'-TGTGGGGCCCTGAAAGGCGAGCTGAGAT-3' (SEQ ID NO: 7) |
| | 5'-ATGGGCCGCCATACGACGACGCTCAACT-3' (SEQ ID NO: 8) |
| ERas | |
| | 5'-ACTGCCCCTCATCAGACTGCTACT-3' (SEQ ID NO: 9) |
| | 5'-CACTGCCTTGTACTCGGGTAGCTG-3' (SEQ ID NO: 10) |
| Nanog | |
| | 5'-AAGCAGAAGATGCGGACTGT-3' (SEQ ID NO: 11) |
| | 5'-ACCACTGGTTTTTCTGCCAC-3' (SEQ ID NO: 12) |
| Oct4 | |
| | 5'-TCTTTCCACCAGGCCCCCGGCTC-3' (SEQ ID NO: 13) |
| | 5'-TGCGGGCGGACATGGGGAGATCC-3' (SEQ ID NO: 14) |
| Rex1 | |
| | 5'-ACGAGTGGCAGTTTCTTCTTGGGA-3' (SEQ ID NO: 15) |
| | 5'-TATGACTCACTTCCAGGGGGCACT-3' (SEQ ID NO: 16) |
| Sox2 | |
| | 5'-TAGAGCTAGACTCCGGGCGATGA-3' (SEQ ID NO: 17) |
| | 5'-TTGCCTTAAACAAGACCACGAAA-3' (SEQ ID NO: 18) |
| Utf1 | |
| | 5'-GGATGTCCCGGTGACTACGTCTG-3' (SEQ ID NO: 19) |
| | 5'-GGCGGATCTGGTTATCGAAGGGT-3' (SEQ ID NO: 20) |
| Gapdh | |
| | 5'-CACCATGGAGAAGGCCGGGG-3' (SEQ ID NO: 21) |
| | 5'-GACGGACACATTGGGGGTAG-3' (SEQ ID NO: 22) |

### Example 5: Study on differentiation potency into three germ layers

This example shows the results studied for maintenance of differentiation potency in mouse cell line EB3 when cultured with the use of various cell-supporting materials.

After EB3 cells were cultured under KSR-supplemented and serum-free conditions in Example 3, the cells were acclimated again under serum conditions by being subcultured for an additional 5 passages under maintenance culture conditions (S+Gl) before use in the differentiation-inducing test. For culture in a serum-free medium, a system of gelatin-coated plates (K+Gl) and a system of recombinant human laminin-5-coated plates (K+Lm5-4 or K+Lm5-2) were used. Experimental groups in which culture in these systems was followed by acclimation under serum conditions are expressed as K+Gl ⇒ S+Gl, K+Lm5-4 ⇒ S+Gl, and K+Lm5-2 ⇒ S+Gl, respectively.

Induction of differentiation was accomplished as follow. The cells were suspended in a LIF (ESGRO)-free maintenance medium to prepare hanging drops (1000 cells/drop), in which embryoid bodies were formed for 2 days. The formed embryoid bodies were transferred to bacterial culture plates and cultured under floating conditions in the LIF-free maintenance medium for an additional 5 days. Figure 7 shows the embryoid bodies observed at that time.

The embryoid bodies after floating culture were transferred to 1 mg/ml Gl-coated chamber slides (NUNC) and cultured for 3 days under adhesion conditions, followed by immunostaining to detect differentiation markers, thereby studying the differentiation potency of ES cells (i.e., differentiation into cells of the endodermal, mesodermal and ectodermal lineages).

In this study, the markers used were α-fetoprotein (AFP) for cells of the endodermal lineage, α-smooth muscle actin (α-SMA) for cells of the mesodermal lineage, and β-III tubulin (tubulin) for cells of the ectodermal lineage. For immunostaining, the cells were fixed with 4% formaldehyde and then blocked with a 5% FBS solution supplemented with 0.1% Triton-X100 (Nacalai). After blocking, the cells were treated with primary and secondary antibodies, stained with DAPI (Nacalai), embedded in VECTASHIELD (VECTOR Laboratories) and then observed under a fluorescent microscope to detect marker expression.

The primary antibodies used in immunostaining were anti-AFP polyclonal antibody (DAKO) for detection of α-fetoprotein, anti-α-SMA monoclonal antibody (DAKO) for detection of α-smooth muscle actin, and anti-β-III tubulin monoclonal antibody (Chemicon) for detection of β-III tubulin. The secondary antibodies used were anti-rabbit IgG polyclonal antibody (Santa Cruz Biotechnology) and anti-mouse IgG polyclonal antibody (Santa Cruz Biotechnology).

In parallel with induction of differentiation in the experimental groups of S+Gl, K+Gl ⇒ S+Gl, K+Lm5-4 ⇒ S+Gl and K+Lm5-2 ⇒ S+Gl as described above, negative controls were prepared for these experimental groups by being cultured in the presence of LIF in a system of normal maintenance culture (S+Gl) without conducting a series of differentiation-inducing processes, including embryoid body formation. These negative controls were also immunostained in the same manner.

In these four experimental groups, differentiation markers AFP, α-SMA and β-III tubulin were compared for their expression between each experimental group induced to differentiate and the corresponding negative control not induced to differentiate (Figures 8 to 11). Figure 8 shows the results obtained for the experimental group of S+Gl, Figure 9 shows the results obtained for the experimental group of K+Gl ⇒ S+Gl, Figure 10 shows the results obtained for the experimental group of K+Lm5-4 ⇒ S+Gl, and Figure 11 shows the results obtained for the experimental group of K+Lm5-2 ⇒ S+Gl.

As a result, in all of the experimental groups, three signals of AFP, α-SMA and β-III tubulin were all observed in the experimental groups induced to differentiate (lower panel of Figure 8, lower panel of Figure 9, lower panel of Figure 10, lower panel of Figure 11). In contrast, three signals of AFP, α-SMA and β-III tubulin were not observed in the negative controls cultured in the system of normal maintenance culture, thus indicating that the cells were not induced to differentiate (upper panel of Figure 8, upper panel of Figure 9, upper panel of Figure 10, upper panel of Figure 11).

This result means that EB3 cells were induced to differentiate only after a series of differentiation-inducing processes and they became differentiated cells of three germ layers expressing AFP, αSMA, β-III tubulin or other markers. Taken together with the results of Example 4, the results of this example suggested that ES cells maintained in the presence of Lm5 not only remained in an undifferentiated state, but also retained pluripotency, i.e., the ability to differentiate into a wide range of cells upon induction.

### Example 6: Preparation of serum replacement of another composition than KSR

A serum replacement of another composition was prepared, whose composition was different from that of KSR mentioned above. The composition of the serum replacement prepared in this example is shown in Table 3.

**[Table 3]**

| Composition of serum replacement whose composition is known | |
|---|---|
| Glycine (Nacalai) | 150 mg/l |
| Histidine (Nacalai) | 940 mg/l |
| Isoleucine (Nacalai) | 3400 mg/l |
| Methionine (Nacalai) | 90 mg/l |
| Phenylalanine (Nacalai) | 1800 mg/l |
| Proline (Nacalai) | 4000 mg/l |
| Hydroxyproline (Nacalai) | 100 mg/l |
| Serine (Nacalai) | 800 mg/l |
| Threonine (Nacalai) | 2200 mg/l |
| Tryptophan (Nacalai) | 440 mg/l |
| Tyrosine (SIGMA) | 77 mg/l |
| Valine (Nacalai) | 2400 mg/l |
| Thiamine (Nacalai) | 33 mg/l |
| Ascorbic acid (SIGMA) | 330 mg/l |
| Reduced glutathione (SIGMA) | 10 mg/l |
| Human transferrin (Nacalai) | 55 mg/l |
| Bovine insulin (SIGMA) | 100 mg/l |
| Sodium selenite (SIGMA) | 0.07 mg/l |
| BSA (Invitrogen) | 83000 mg/l |
| AgNO₃ (Mediatech Inc.) | 0.0017 mg/l |
| AlCl₃·6H₂O (Mediatech Inc.) | 0.012 mg/l |
| Ba(C₂H₃O₂)₂ (Mediatech Inc.) | 0.0255 mg/l |
| CdCl₂ (Mediatech Inc.) | 0.0228 mg/l |
| CoCl₂·6H₂O (Mediatech Inc.) | 0.0238 mg/l |
| Cr₂Cl₃ (Mediatech Inc.) | 0.0032 mg/l |
| GeO₂ (Mediatech Inc.) | 0.0053 mg/l |
| KBr (Mediatech Inc.) | 0.0012 mg/l |
| KI (Mediatech Inc.) | 0.0017 mg/l |
| MnSO₄·H₂O (Mediatech Inc.) | 0.0017 mg/l |
| NaF (Mediatech Inc.) | 0.042 mg/l |
| Na₂SiO₃·9H₂O (Mediatech Inc.) | 1.4 mg/l |
| NH₄VO₃ (Mediatech Inc.) | 0.0065 mg/l |
| (NH₄)₆Mo₇O₂₄·4H₂O (Mediatech Inc.) | 0.0124 mg/l |
| NiSO₄·6H₂O (Mediatech Inc.) | 0.0013 mg/l |
| RbCl (Mediatech Inc.) | 0.0121 mg/l |
| SnCl₂ (Mediatech Inc.) | 0.0012 mg/l |
| ZrOCl₂·8H₂O (Mediatech Inc.) | 0.0322 mg/l |

In distilled water, the various amino acids shown in Table 3 (glycine, histidine, isoleucine, methionine, phenylalanine, methionine, phenylalanine, proline, hydroxyproline, serine, threonine, tryptophan, tyrosine, valine) were dissolved at 3-fold concentrations to prepare a 3-fold concentrated amino acid solution. To this 3-fold concentrated amino acid solution, thiamine, ascorbic acid and reduced glutathione were each added in an amount required to give a 3-fold concentration. This solution is designated as solution A. Next, human transferrin and BSA were dissolved at 2-fold concentrations in distilled water to prepare a 2-fold concentrated BSA solution. This solution is designated as solution B. Further, sodium selenite was dissolved in a required amount in distilled water to prepare a 7% sodium selenite solution. This solution is designated as solution C. To the solutions A, B and C thus prepared, a bovine insulin solution (SIGMA) and trace metal elements were added to prepare the serum replacement of this example. It should be noted that the trace metal elements added here are commercially available products, Trace Elements B and C (Mediatech Inc.) whose composition is known. The serum replacement prepared in this example is not commercially available, but its composition is known.

### Example 7: Study using medium supplemented with serum replacement of Example 6

In this example, the serum replacement prepared in Example 6 was added to GMEM as an alternative to serum, as in the case of KSR, and the resulting medium was used as a maintenance medium for culture of mouse ES cells. The medium thus prepared is designated as medium Y. Using medium Y, proliferation assay was performed as described in Example 3. Further, undifferentiation markers were detected as described in Example 4. Furthermore, differentiation potency into three germ layers was studied as described in Example 5.

### (1) Proliferation assay

The results of proliferation assay using medium Y are shown in Figure 12. The experiment was performed in the same manner as shown in Example 3, except that medium Y was used as a maintenance medium. As a result of proliferation assay using mouse ES cell line EB3, the experimental groups where the cells were cultured on rLm5-coated plates in medium Y (Y+Lm5-4, Y+Lm5-2) were found to show a proliferative effect on mouse ES cells. In addition, the use of Lm5 resulted in better proliferation, when compared to the experimental groups using other extracellular matrixes (Gl, Lm-Mix, Mg).

### (2) Detection of undifferentiation markers

After proliferation assay as described above, undifferentiation markers were studied by RT-PCR for their expression in each experimental group. The experiment was performed in the same manner as shown in Example 4, except that medium Y was used as a maintenance medium. Figure 13 shows the results of 7 undifferentiation markers detected in the same manner as shown in Example 4. In the case of using medium Y for culture of mouse ES cells, all the undifferentiation markers were also confirmed to be expressed in the experimental groups where the cells were cultured on rLm5-coated plates (Y+Lm5). Thus, it was suggested that EB3 cells retained their undifferentiated state even when subcultured for 10 or more passages in a system of rLm5 using medium Y.

### (3) Study on differentiation potency into three germ layers

A differentiation-inducing test was also performed in the same manner as shown in Example 5, except that medium Y was used as a maintenance medium. All of the experimental groups induced to differentiate were found to form embryoid bodies morphologically closely resembling those observed in the control group (S+Gl). The results obtained are shown in Figure 14.

After induction of differentiation, markers for differentiation into three germ layers (AFP, α-SMA, β-III tubulin) were detected. In the experimental groups of S+Gl (Figure 15), K+Gl ⇒ S+Gl (Figure 16), Y+G1 ⇒ S+Gl (Figure 17), Y+Lm5-4 ⇒ S+Gl (Figure 18), Y+Lm5-2 ⇒ S+Gl (Figure 19), Y+Lm-Mix ⇒ S+Gl (Figure 20) and Y+Mg ⇒ S+Gl (Figure 21), the differentiation markers were studied for their expression between each experimental group induced to differentiate (lower panel, LIF-) and the corresponding negative control not induced to differentiate (upper panel, LIF+).

As shown in lower panels (LIF-) of Figures 15 to 21, immunostaining after induction of differentiation showed the expression of all three markers AFP, α-SMA and β-III tubulin in each experimental group. In contrast, as shown in the upper panels (LIF+) of Figures 15 to 21, no expression of AFP, α-SMA and β-III tubulin was observed in the negative controls cultured in a system of normal maintenance culture without induction of differentiation, thus indicating that the cells were not induced to differentiate.

The above results suggested that in the case of using medium Y, rLm5 also supported the proliferation of mouse ES cells, and the proliferated mouse ES cells also retained their undifferentiated state. This indicated that rLm5 may serve as a useful supporting material for mouse ES cells under feeder cell-free and serum-free conditions.

### Example 8: Adhesion assay using human iPS cells

This example shows the results of adhesion assay using human iPS cells, obtained with the use of various cell-supporting materials.

The human iPS cells used were those of cell line 201B2 or 201B7 established by the Department of Stem Cell Biology, Institute for Frontier Medical Sciences, Kyoto University. In Figure 22, the left panel shows the morphology of cell line 201B2, while the right panel shows the morphology of cell line 201B7.

Human iPS cells were cultured for 1 hour in the presence of 10 µM Y-27632 (WAKO) before being released from the dishes, and then used in the experiment. It should be noted that the same treatment was also performed in the experiments of Example 8 and the subsequent examples where human iPS cells were used.

For maintenance of human iPS cells, the supernatant of mouse embryonic fibroblasts prepared in DMEM/F12 medium containing 20% KSR, 2 mM glutamine, 1% nonessential amino acids and 10⁻⁴ M 2-mercaptoethanol was supplemented with 4 ng/ml bFGF (WAKO) and used as a maintenance medium (MEF-CM).

The cell-supporting materials used were mitomycin C-treated SNL feeder cells (Fd) as well as various extracellular matrixes (Gl, Lm5, Mg, Vn, Co, Fn, Lm2, Lm-Mix). Further, plates were prepared by being treated with these respective cell-supporting materials, and were subjected to adhesion assay for each cell-supporting material using human iPS cell line 201B7, as described below.

96-well plates (Corning) were treated with various extracellular matrix proteins prepared at desired concentrations, and then blocked with a 1.2% BSA (SIGMA) solution at 37°C for 1 hour. The various extracellular matrix proteins were prepared at the following concentrations: 5 mg/ml and 1.25 mg/ml for Gl, 50 µg/ml and 12.5 µg/ml for Lm2, 50 µg/ml and 12.5 µg/ml for Lm-Mix, 500 µg/ml and 125 µg/ml for Mg, 50 µg/ml and 12.5 µg/ml for Co, 50 µg/ml and 12.5 µg/ml for Fn, and 50 µg/ml and 12.5 µg/ml for Vn. rLm5 was prepared by two-fold serial dilution at five concentrations: 50 µg/ml, 25 µg/ml, 12.5 µg/ml, 6.25 µg/ml and 3.125 µg/ml. Moreover, an additional experimental group (Lm5+Co) was also prepared by being treated with both 25 µg/ml Lm5 and 50 µg/ml Co.

Human iPS cells were treated with trypsin and dispersed into single cells, and then seeded at 20000 cells/well in the plates and cultured at 37°C under gas phase conditions of 5% CO₂ and 95% air for 60 minutes. After culture, the plates were tapped gently to release less adhered cells from the plate surface, followed by treatment with Percoll (GE healthcare) to remove these cells. The adhered cells were fixed with 25% glutaraldehyde (Nacalai) and stained with 2.5% crystal violet (Nacalai) for comparison of relative cell counts.

Figure 23 shows the results evaluated for the effect of the various extracellular matrix proteins on human iPS cell adhesion after culture for 60 minutes, as analyzed by OD595 measurement. Further, Figure 24 shows the morphology of cells after the assay in each experimental group.

In Figure 23, the experimental groups of rLm5 were found to show higher OD595 values than the experimental groups of the other extracellular matrix proteins, indicating that rLm5 showed strong adhesion activity on human iPS cells. Also in Figure 24, many adhered cells were observed in the experimental groups of rLm5. Moreover, rLm5 was found to exert stronger adhesion activity when used in combination with Co (rLm5+Co) than when used alone.

### Example 9: Colony assay using human iPS cells

This example shows the results of colony assay using human iPS cell line 201B2, obtained with the use of various cell-supporting materials.

The cell-supporting materials used were Gl, Lm5, Mg, Vn, Co, Fn, Lm2 and Lm-Mix. Further, 60 mm dishes (IWAKI) were prepared by being treated with these respective cell-supporting materials, and were subjected to colony assay for each cell-supporting material, as described below.

The various extracellular matrix proteins were prepared at the following concentrations: 1 mg/ml for Gl, 30 µg/ml, 15 µg/ml, 8 µg/ml, 4 µg/ml and 2 µg/ml for Lm5, 30 µg/ml, 15 µg/ml, 8 µg/ml, 4 µg/ml and 2 µg/ml for Lm2, 30 µg/ml, 15 µg/ml, 8 µg/ml, 4 µg/ml and 2 µg/ml for Lm-Mix, 300 µg/ml for Mg, 30 µg/ml for Co, 30 µg/ml for Fn, and 10 µg/ml for Vn.

In this example, human iPS cells were prepared in a state of single cells dispersed by trypsin treatment (hereinafter referred to as "Single") and in a state of cell clumps prepared by gentle pipetting while preventing dispersion (hereinafter referred to as "Clump"), both of which were subjected to the assay. Namely, human iPS cells were seeded in Single or Clump state at a density corresponding to 1000 cells per 60 mm dish. Culture was performed at 37°C under gas phase conditions of 5% CO₂ and 95% air for 13 days (Single) or 6 days (Clump). After culture, the number of formed colonies was counted for each case.

The results obtained are shown in Figure 25. In Figure 25, the upper panel shows the results obtained for Single, while the lower panel shows the results obtained for Clump.

In Single, colony formation was observed in the experimental groups of rLm5, Lm-Mix, Vn and Mg, and the number of colonies formed in the experimental group of 8 µg/ml Lm5 or 30 µg/ml Lm-Mix was close to that in the positive control, i.e., the experimental group using SNL feeder cells (Fd). On the other hand, in Clump, colony formation was observed in the experimental groups of rLm5, Lm2, Lm-Mix, Vn, Mg and Co, and the experimental groups of rLm5 showed colony formation comparable to or better than that observed in Fd.

Further, the formed colonies were immunostained to detect a marker of human pluripotent stem cells, thereby evaluating whether the cultured human iPS cells were in an undifferentiated state. The marker of human pluripotent stem cells used for this purpose was a cell surface antigen marker, SSEA3.

For immunostaining, the cells were fixed with 4% formaldehyde and then blocked with 1% BSA-containing PBS. After blocking, the cells were treated with primary and secondary antibodies, stained with Hoechist 33342 (Invitrogen) and then observed under a fluorescent microscope to detect marker expression. The primary and secondary antibodies used in immunostaining were anti-SSEA3 monoclonal antibody and anti-rat IgM antibody (Jackson ImmunoResearch), respectively.

The results of immunostaining are shown in Figure 26. Figure 26A shows the results of Single, while Figure 26B shows the results of Clump. The ES cell marker SSEA3 was detected in colonies formed from both Single and Clump. This suggested that colonies formed from human iPS cells retained their undifferentiated state.

The above results suggested that rLm5 supported the colony formation of human iPS cells comparably to or better than the other extracellular matrixes, and the formed colonies also retained their undifferentiated state.

### Example 10: Maintenance culture test using human iPS cells

This example shows the results of maintenance culture test using human iPS cell line 201B7, obtained with the use of various cell-supporting materials.

The cell-supporting materials used were G1, Lm5, Lm2, Vn, Co and Fn. Further, 6-well plates (Falcon) were prepared by being treated with these respective cell-supporting materials, and were subjected to colony assay for each cell-supporting material, as described below. The maintenance medium used was MEF-CM as mentioned in Example 8.

The various extracellular matrix proteins were prepared at the following concentrations: 1 mg/ml for G1, 2 µg/ml for Lm5, 30 µg/ml for Lm2, 30 µg/ml for Co, 30 µg/ml for Fn, and 10 µg/ml for Vn.

In this example, human iPS cells were assayed only in Clump state. Once a week, 1/9 of all the cells were re-seeded in newly prepared 6-well plates treated with the same various extracellular matrixes. The cells were cultured at 37°C under gas phase conditions of 5% CO₂ and 95% air for 5 weeks. Figure 27 shows the morphology of cells cultured for 5 weeks.

As a result, in the experimental groups of the various extracellular matrix proteins tested, cell proliferation was observed upon culture for 1 week after subculture at a level comparable to that in the positive control, i.e., the experimental group of SNL feeder cells (Fd). As shown in Figure 27, this tendency was also observed after culture for 5 weeks. This result indicated that rLm5 and Fd were able to almost equally support the proliferation of human iPS cells. In the case of Lm2, colony formation disappeared during the course of culture.

### Example 11: Detection of undifferentiation markers in human iPS cells

In this example, NANOG, OCT4 and SOX2, which are know as undifferentiation markers of human pluripotent stem cells, were detected by RT-PCR on their genes to study whether rLm5 had the effect of allowing human iPS cells to remain in an undifferentiated state.

From the human iPS cells subcultured for 5 weeks in Example 10, total RNA was extracted using TRIZOL (Invitrogen). After extraction, a ThermoScript RT-PCR System (Invitrogen) was used to synthesize cDNA by reverse transcription reaction. The synthesized cDNA was used as a template to perform PCR with the primers shown in Table 4. Each gene was denatured at 94°C for 10 seconds, annealed for 15 seconds, and elongated at 72°C for 30 seconds. Annealing was performed at a temperature of 60°C for OCT4 and GAPDH, and 55°C for NANOG and SOX2.

**[Table 4]**

| | RT-PCR primers |
|---|---|
| OCT4 | |
| | 5'-GACAGGGGGAGGGGAGGAGCTAGG-3' (SEQ ID NO: 23) |
| | 5'-CTTCCCTCCAACCAGTTGCCCCAAAC-3' (SEQ ID NO: 24) |
| NANOG | |
| | 5'-CAGCCCTGATTCTTCCACCAGTCCC-3' (SEQ ID NO: 25) |
| | 5'-TGGAAGGTTCCCAGTCGGGTTCACC-3' (SEQ ID NO: 26) |
| SOX2 | |
| | 5'-GGGAAATGGGAGGGGTGCAAAAGAGG-3' (SEQ ID NO: 27) |
| | 5'-TTGCGTGAGTGTGGATGGGATTGGTG-3' (SEQ ID NO: 28) |
| GAPDH | |
| | 5'-GTGGACCTGACCTGCCGTCT-3' (SEQ ID NO: 29) |
| | 5'-GGAGGAGTGGGTGTCGCTGT-3' (SEQ ID NO: 30) |

The results of RT-PCR are shown in Figure 28. All of the experimental groups showed the expression of the three tested factors. This suggested that when cultured using rLm5 as a cell-supporting material, human iPS cells retained their undifferentiated state.

### Example 12: Differentiation-inducing test in human iPS cells

This example shows the results studied for maintenance of differentiation potency in human iPS cells when cultured with the use of various cell-supporting materials. In human iPS cells induced to differentiate, differentiation markers were detected by RT-PCR to study whether human iPS cells cultured with the use of various cell-supporting materials retained differentiation potency.

After culture for 3 weeks, i.e., during the third passage in Example 10, a part of the cells were collected and used in the differentiation-inducing test. Induction of differentiation was accomplished as follow.

Clumps, which had been prepared from human iPS cells under culture by being released from the plates, were cultured using low adsorption plates (NUNC) under floating conditions for 8 days in DMEM/F12 medium supplemented with 20% KSR, 2 mM glutamine, 1% nonessential amino acids and 10⁻⁴ M 2-mercaptoethanol. The embryoid bodies thus formed were re-seeded in 1 mg/ml Gl-coated 6-well plates (Falcon) and cultured under adhesion conditions for an additional 8 days.

Figure 29 shows the morphology of cells after adhesion culture. The cells after being induced to differentiate in the bFGF-free medium exhibit various morphologies, which suggests that the cells have differentiated. The cell morphologies observed in this study clearly differ from those observed during normal maintenance culture in the presence of bFGF, as shown in Figure 27.

From the human iPS cells induced to differentiate, RNA was extracted and cDNA was synthesized in the same manner as shown in Example 11, and the synthesized cDNA was used as a template to perform PCR with the primers shown in Table 5. The markers used were SOX17 and AFP as endodermal markers, BRACHYURY and MSX1 as mesodermal markers, PAX6 as an ectodermal marker, and CDX2 as a trophectodermal marker. Each gene was denatured at 94°C for 10 seconds, annealed for 10 seconds, and elongated at 72°C for 30 seconds. Annealing was performed at a temperature of 63°C for SOX17, BRACHYURY, MSX1 and PAX6, 65°C for AFP, and 55°C for CDX2. Only in the case of CDX2, elongation was performed at 72°C for 15 seconds.

The results of RT-PCR are shown in Figure 30. The experimental group of rLm5 showed the expression of all the six tested factors, as in the case of the positive control, i.e., the experimental group of Fd. Thus, it is suggested that rLm5 had the effect of maintaining pluripotency on human iPS cells. In contrast, the experimental groups of some other extracellular matrixes (e.g., Vn, Co) were found to show very low expression of the mesodermal marker MSX1. This indicated that these extracellular matrixes may cause human iPS cells to partially lose their differentiation potency or may induce resistance to differentiation.

**[Table 5]**

| | RT-PCR primers |
|---|---|
| SOX17 | |
| | 5'-CGCTTTCATGGTGTGGGCTAAGGACG-3' (SEQ ID NO: 31) |
| | 5'-TAGTTGGGGTGGTCCTGCATGTGCTG-3' (SEQ ID NO: 32) |
| AFP | |
| | 5'-GAATGCTGCAAACTGACCACGCTGGAAC-3' (SEQ ID NO: 33) |
| | 5'-TGGCATTCAAGAGGGTTTTCAGTCTGGA-3' (SEQ ID NO: 34) |
| BRACHYURY | |
| | 5'-GCCCTCTCCCTCCCCTCCACGCACAG-3' (SEQ ID NO: 35) |
| | 5'-CGGCGCCGTTGCTCACAGACCACAGG-3' (SEQ ID NO: 36) |
| MSX1 | |
| | 5'-CGAGAGGACCCCGTGGATGCAGAG-3' (SEQ ID NO: 37) |
| | 5'-GGCGGCCATCnCAGCTTCTCCAG-3' (SEQ ID NO: 38) |
| PAX6 | |
| | 5'-ACCCATTATCCAGATGTGTTTGCCCGAG-3' (SEQ ID NO: 39) |
| | 5'-ATGGTGAAGCTGGGCATAGGCGGCAG-3' (SEQ ID NO: 40) |
| CDX2 | |
| | 5'-GCAGAGCAAAGGAGAGGAAA-3' (SEQ ID NO: 41) |
| | 5'-CAGGGACAGAGCCAGACACT-3' (SEQ ID NO: 42) |

The present invention allowed pluripotent stem cells to proliferate in an undifferentiated state, without the need to use feeder cells or serum, when culturing them in a system containing laminin-5, an extracellular matrix molecule. According to the method of the present invention, pluripotent stem cells can be cultured without using any animal-derived material such as feeder cells or serum, which eliminates risks of immunological rejection, virus infection and so on. Because of their totipotency, human-derived pluripotent stem cells have a great potential to be used as cellular materials in regenerative medicine. In particular, human induced pluripotent stem cells (iPS cells) have no ethical problem and are also free from the problem of immunological rejection because they can be prepared from patients' own cells.

### SEQUENCE LISTING FREE TEXT

<SEQ ID NO: 1>
   SEQ ID NO: 1 shows the nucleotide sequence of human laminin α3 chain.
<SEQ ID NO: 2>
   SEQ ID NO: 2 shows the amino acid sequence of human laminin α3 chain.
<SEQ ID NO: 3>
   SEQ ID NO: 3 shows the nucleotide sequence of human laminin β3 chain.
<SEQ ID NO: 4>
   SEQ ID NO: 4 shows the amino acid sequence of human laminin β3 chain.
<SEQ ID NO: 5>
   SEQ ID NO: 5 shows the nucleotide sequence of human laminin γ2 chain.
<SEQ ID NO: 6>
   SEQ ID NO: 6 shows the amino acid sequence of human laminin γ2 chain.
<SEQ ID NOs: 7 to 22>
   SEQ ID NOs: 7 to 22 show the nucleotide sequences of RT-PCR primers used for undifferentiation marker detection in ES cells.
<SEQ ID NOs: 23 to 30>
   SEQ ID NOs: 23 to 30 show the nucleotide sequences of RT-PCR primers used for undifferentiation marker detection in human iPS cells.
<SEQ ID NOs: 31 to 42>
   SEQ ID NOs: 31 to 42 show the nucleotide sequences of RT-PCR primers used for differentiation marker detection in human iPS cells.

## Claims

1. A method for proliferation of pluripotent stem cells, which comprises culturing the pluripotent stem cells in a medium free from both feeder cells and serum in a system containing laminin-5, wherein the pluripotent stem cells are induced pluripotent cells or embryonic stem cells.

2. The method according to claim 1, wherein the pluripotent stem cells are human induced-pluripotent stem cells or human embryonic stem cells.

3. The method according to claim 1 or claim 2, wherein the culture medium comprises a serum replacement.

4. The method according to claim 3, wherein the serum replacement comprises one or more amino acids selected from the group consisting of glycine, histidine, isoleucine, methionine, phenylalanine, proline, hydroxyproline, serine, threonine, tryptophan, tyrosine and valine, vitamin(s) consisting of thiamine and/or ascorbic acid, one or more trace metal elements selected from the group consisting of silver, aluminum, barium, cadmium, cobalt, chromium, germanium, manganese, silicon, vanadium, molybdenum, nickel, rubidium, tin and zirconium, one or more halogen elements selected from the group consisting of bromine, iodine and fluorine, as well as one or more ingredients selected from the group consisting of albumin, reduced glutathione, transferrin, insulin and sodium selenite.

5. The method according to any one of claims 1 to 4, wherein the pluripotent stem cells are cultured in a system containing laminin-5 and (an) additional extracellular matrix protein(s).

6. The method according to claim 5, wherein the additional extracellular matrix protein is collagen.

7. The method according to any one of claims 1 to 6, wherein the pluripotent stem cells are cultured in a laminin-5-treated culture vessel.

8. The method according to claim 7, wherein the laminin-5 is human laminin-5.

9. The method according to any one of claims 1 to 8, wherein the pluripotent stem cells do not differentiate during culture.

10. Use of laminin-5 as a cell-supporting material for a proliferation of induced pluripotent stem cells or embryonic stem cells in a medium free from serum.

## Patentansprüche

1. Verfahren zur Proliferation pluripotenter Stammzellen, umfassend Züchten der pluripotenten Stammzellen in einem Medium, frei von sowohl Beschickerzellen als auch Serum, in einem Laminin-5 enthaltenden System, wobei die pluripotenten Stammzellen induzierte pluripotente Stammzellen oder embryonische Stammzellen sind.

2. Verfahren gemäß Anspruch 1, wobei die pluripotenten Stammzellen menschliche induziert pluripotente Stammzellen oder menschliche embryonische Stammzellen sind.

3. Verfahren gemäß Anspruch 1 oder Anspruch 2, wobei das Zuchtmedium einen Serum-Ersatz umfasst.

4. Verfahren gemäß Anspruch 3, wobei der Serum-Ersatz eine oder mehrere Aminosäuren umfasst, ausgewählt aus der Gruppe Glycin, Histidin, Isoleucin, Methionin, Phenylalanin, Prolin, Hydroxyprolin, Serin, Threonin, Tryptophan, Tyrosin und Valin, Vitamin(e), bestehend aus Thiamin und/oder Ascorbinsäure, ein oder mehrere Spurenmetallelemente, ausgewählt aus der Gruppe Silber, Aluminium, Barium, Cadmium, Cobalt, Chrom, Germanium, Mangan, Silicium, Vanadium, Molybdän, Nickel, Rubidium, Zinn und Zirkon, ein oder mehrere Halogenelemente, ausgewählt aus der Gruppe Brom, lod und Fluor, sowie eine oder mehrere Zutaten, ausgewählt aus der Gruppe Albumin, reduziertes Glutathion, Transferrin, Insulin und Natriumselenit.

5. Verfahren gemäß irgendeinem der Ansprüche 1 bis 4, wobei die pluripotenten Stammzellen in einem Laminin-5 und ein oder mehrere zusätzliche extrazelluläre Matrixeiweiße enthaltenden System gezüchtet werden.

6. Verfahren gemäß Anspruch 5, wobei das zusätzliche extrazelluläre Matrixeiweiß Collagen ist.

7. Verfahren gemäß irgendeinem der Ansprüche 1 bis 6, wobei die pluripotenten Stammzellen in einem mit Laminin-5 behandelten Zuchtgefäß gezüchtet werden.

8. Verfahren gemäß Anspruch 7, wobei das Laminin-5 menschliches Laminin-5 ist.

9. Verfahren gemäß irgendeinem der Ansprüche 1 bis 8, wobei die pluripotenten Stammzellen sich nicht während der Zucht differenzieren.

10. Verwendung von Laminin-5 als Zellträgermaterial für eine Proliferation von induzierten pluripotenten Stammzellen oder embryonischen Stammzeilen in einem Medium, frei von Serum.

## Revendications

1. Un procédé de prolifération de cellules souches pluripotentes qui comprend la culture des cellules souches pluripotentes dans un milieu exempt à la fois de cellules nourricières et de sérum dans un système contenant de la laminine-5, où les cellules souches pluripotentes sont des cellules pluripotentes induites ou des cellules souches embryonnaires.

2. Le procédé selon la revendication 1, où les cellules souches pluripotentes sont des cellules souches pluripotentes induites humaines ou des cellules souches embryonnaires humaines.

3. Le procédé selon la revendication 1 ou 2, où le milieu de culture contient un substitut de sérum.

4. Le procédé selon la revendication 3, où le substitut de sérum contient un ou plusieurs acides aminés sélectionnés dans le groupe se composant de glycine, histidine, isoleucine, méthionine, phénylalanine, proline, hydroxyproline, sérine, thréonine, tryptophane, tyrosine et valine, des vitamines se composant de thiamine et/ou d'acide ascorbique, un ou plusieurs éléments de métal-trace sélectionnés dans le groupe se composant d'argent, aluminium, baryum, cadmium, cobalt, chrome, germanium, manganèse, silice, vanadium, molybdène, nickel, rubidium, étain et zircone, un ou plusieurs éléments halogène sélectionnés dans le groupe se composant de brome, iode et fluor, ainsi qu'un ou plusieurs ingrédients sélectionnés dans le groupe se composant d'albumine, glutathione réduite, transferrine, insuline et sélénite de sodium.

5. Le procédé selon l'une quelconque des revendications 1 à 4, où les cellules souches pluripotentes sont cultivées dans un système contenant de la laminine-5 et une ou des protéines matricielles extraceliulaires additionnelles.

6. Le procédé selon la revendication 5, où la protéine matricielle extracellulaire additionnelle est collagène.

7. Le procédé selon l'une quelconque des revendications 1 à 6, où les cellules souches pluripotentes sont cultivées dans un récipient de culture traité à la laminine-5.

8. Le procédé selon la revendication 7, où la laminine-5 est de la laminine-5 humaine.

9. Le procédé selon l'une quelconque des revendications 1 à 8, où les cellules souches pluripotentes ne se différencient pas au cours de la culture.

10. L'utilisation de laminine-5 en tant que matériau de support de cellule destiné à une prolifération de cellules souches pluripotentes induites ou de cellules souches embryonnaires dans un milieu exempt de sérum.
